# EUROPEAN PATENT SPECIFICATION

(11) **EP 1 750 792 B1**
(45) Date of publication and mention of the grant of the patent: **10.04.2013**
(21) Application number: 05746464.6
(22) Date of filing: 04.05.2005
(51) Int. Cl.: A61M 5/00

(54) **METHOD AND SYSTEM FOR IMPLEMENTING A GRAPHICAL USER INTERFACE FOR A MULTI-FLUID INJECTION DEVICE**
VERFAHREN UND SYSTEM ZUR IMPLEMENTIERUNG EINER GRAPHISCHEN BENUTZERSCHNITTSTELLE FÜR EINE MEHRFLUIDINJEKTIONSVORRICHTUNG
PROCEDE ET SYSTEME DE REALISATION D'UNE INTERFACE UTILISATEUR GRAPHIQUE POUR UN DISPOSITIF D'INJECTION MULTIFLUIDE

(30) Priority: 04.05.2004 US 568213 P
(43) Date of publication of application: 14.02.2007
(73) Proprietor: Acist Medical Systems, Inc., Eden Prairie, MN 55344 (US)
(72) Inventor: WILLIAMS, Robert, C., Jr., Fort Salonga, NY 11768 (US); GRIFFIN, William, Brightwaters, NY 11718 (US)
(74) Representative: Kinsler, Maureen Catherine
(86) International application number: PCT/US2005/015718
(87) International publication number: WO 2005/107419

(56) References cited:
- EP-A1- 1 402 910
- JP-A- 6 277 283
- US-A- 4 562 829
- US-A- 5 221 268
- US-B1- 6 643 537
- US-B2- 6 692 457

## Description

### BACKGROUND OF THE INVENTION

The present invention relates generally to medical devices for injecting a fluid into a subject and more particularly to a user interface for selecting and controlling injection parameters.

Some available diagnostic imaging equipment (e.g., CT scanners, PET apparatus, MRI apparatus, etc) may rely on injecting a fluid, such as a contrast media, into a subject to obtain a diagnostic image. Fluid injection devices have been developed that may be used to automatically administer contrast media to a subject. Such fluid injection systems may rely upon a user to determine the injection parameters such as the flow rate of the fluid, volume of the fluid to be injected, duration of injection, and the like. The operator may then enter the injection parameters into the fluid injection device so that the fluid injection device may inject a contrast media into a subject based on the inputted injection parameters. The injected contrast media is then physiologically processed by the subject, permitting the imaging equipment to obtain an enhanced image of the subject.

In some cases, it may be desirable to inject multiple fluids into a subject in series or concurrently. To inject multiple fluids into a subject, a user may enter injection parameters for injecting a first fluid into a subject and injection parameters for injecting a second fluid into the subject. The operator may also specify at which time the second injection may begin. During the course of an injection function, it may be desirable to vary the injection parameters, such as flow rate or volume, for a given fluid to improve the quality of images obtained. In some cases, a fluid injection function may be divided into one or more injection phases. An injection phase has traditionally been described as a constant injection flow rate for a fixed volume of fluid. A series of injection phases for a given fluid may be grouped together into an injection protocol.

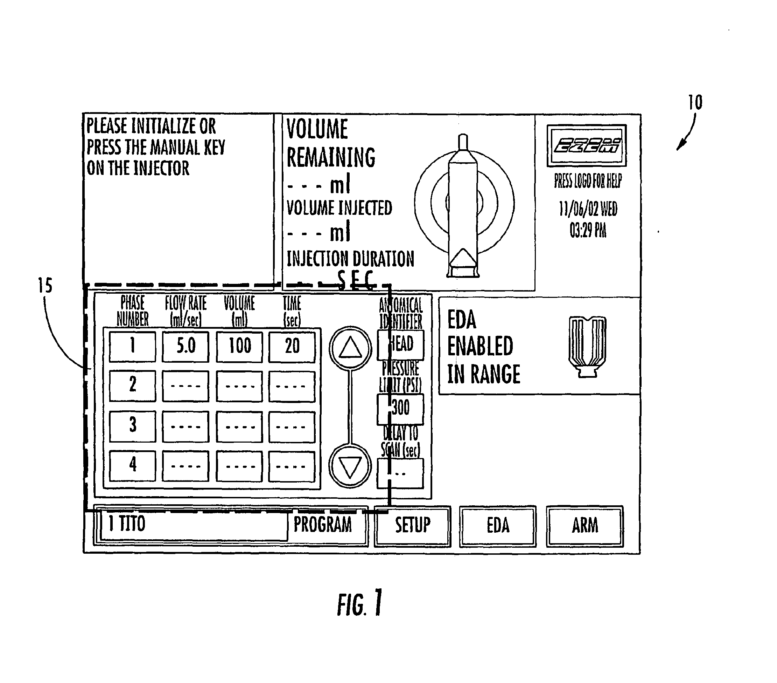

By way of background, FIG. 1 illustrates an EMPOWER CT remote control interface 10 having a display region 15 used to both sequentially list and specify phases of constant flow rate and fixed volume separately. Display region 15 is highlighted by the dashed rectangle. Limitations and restrictions of this interface may include: (i) the flow rate and volume fields are individually touch/mouse activated for input or editing purposes; and (ii) once touch/mouse activated, numeric values for individual flow rate and volume fields are specified via a numeric key-pad or up-down arrows. In some cases, there may be a practical limitation on number of phases that may be displayable in display region 15 and it may not be possible to concurrently visualize all phases that may be desirable in an injection protocol. Prior devices may rely on up/down scroll arrows to permit a user to view injection phases that are not shown on the screen.

In a multi-fluid injection procedure, the above described display region may need to be expanded to include data for one or more additional fluids. The additional data may be used to specify fluid type on a per phase basis, e. g., contrast, saline, or flushing medium, in addition to constant flow rate and fixed volume.

U. S. Patent Application No. 2004/0199076 to Nemoto describes a liquid injector having an integrated control panel that may permit a user to use a touchpen to graphically input a desired flow rate and injection time into a computer. The liquid inj ector may then use the graph to inject a liquid into a subject at the desired flow rate and length of time. Although the inj ector device described by Nemoto overcomes some of the disadvantages described above, It still has several disadvantages. First, the control panel is integrated into the injection device. This may limit the placement of the injection device and may require a user to stay in an imaging room until the injection has been completed. Second, to graphically input a desired flow rate and injection time, a user first selects a body region to be injected. In some applications, it may not be desirable to select a particular body region when inputting the desired injection parameters.

U.S. Patent No. 6,643,537 entitled 'Programmable Injector Control' to Zatezalo *et al*. is directed to a fluid injection programming capability where phases of contrast medium injection and flushing medium injection can be selectively ordered in a number of different protocols.

### BRIEF SUMMARY OF THE INVENTION

Various aspects of the invention are defined in the independent claims. Some preferred features are defined in the dependent claims.

In one alternative embodiment, the invention is directed to a graphical user interface having a graph space in which a user may create a graph that may be used to control the injection behavior of a fluid injection device. In some embodiments, a user may use the graph space to graphically plot a graph that may be used to control the behavior of a fluid injection device. In some embodiments, a computer or control console may use the graph to define an injection protocol for injecting a fluid into a subject.

In one alternative embodiment, the invention comprises a graphical visualization tool for controlling the behavior of a fluid injection device using a graphical user interface. The graphical visualization tool may comprise a graph space window for inputting and displaying a desired injection function for a fluid, one or more fluid selector icons that may permit a user to select a fluid. and a location selection icon that may permit a user to navigate within the graph space to create an injection function for a selected fluid, whereby a fluid injection device may use the created injection function to inject a fluid into a subject. In some embodiments, one or more injection functions may be graphically plotted within the same graph space to define an injection protocol.

In one embodiment, the graphical user interface may Include a graphical visualization tool that may be used to graphically plot an injection function in a graph space that may be used to control the injection behavior of a fluid injection device. In another alternative embodiment, the graphical visualization tool may include a control panel that may have one or more of: fluid selector icons, location selector icon, and a segment selector icon. In some embodiments, the one or more fluid selector icons may permit a user to select one or more fluid for which to create an injection function in the graph space. In one embodiment, the location selector icon may permit a user to navigate within the graph space and select an nth point and an nth + 1 point that may be connected together to define a piecewise injection protocol within the graph space.

In one alternative embodiment, the invention may include a graphical user interface having a graph space in which a user may graphically plot one or more injection functions for one or more fluids. In some embodiments one or more injection functions may be created wherein a portion of one injection function overlaps at least a portion of a second injection function within the graph space. The overlapping portions of the injection functions may define an injection protocol wherein two or more fluids may be injected concurrently.

In one alternative embodiment, the invention may comprise a fluid injection system that may include a fluid injection device, a computer that may be operably connected to the fluid injection device. In some embodiments, the computer may have a computer readable code thereon for enabling a processor to control the fluid injector device and to permit a user to graphically input fluid injection parameters for one or more fluids into the computer using a graphical user interface. In one alternative embodiment, a user may graphically input fluid injection parameters into the computer independently of the body region of a subject to be injected. The fluid injection system may also include a visual display for displaying the graphical user interface, and at least one input device that may permit a user to graphically input the injection parameters on the graphical user interface. The computer may then use the fluid injection parameters that have been graphically plotted using the graphical user interface to control the behavior of the fluid injection device. In some embodiments, the inputted injection parameters may correspond to an injection function or injection protocol.

In another alternative embodiment, the invention may comprise a computer program product that may include a computer usable medium having computer readable program code embodied therein that may be configured to control a fluid injection device. In some embodiments, the computer program product may include computer readable code configured to cause a computer to display a graphical user interface that may be used to graphically plot an injection function. In some embodiments, the graphical user interface may include a graph space that defines an area for graphically plotting an injection function and that may be used by a computer to control the behavior of a fluid injection device. In some embodiments, the computer program product may include one or more fluid selector icons that may permit a user to select one or more fluids for which to graphically plot an injection function in the graph space, and a location selection icon that may permit a user to navigate within graph space to graphically plot an injection function in the graph space for the selected fluid. In one embodiment, the computer readable code may be configured to cause a computer to control the fluid injection device using the injection function.

In yet another embodiment, the invention may comprise a method for controlling a fluid injection device comprising the steps of: a) presenting a graphical user interface to a user wherein the graphical user interface may include a graph space window for inputting and displaying an injection function for a fluid; b) selecting a first fluid; c) selecting a first point in the graph space; d) selecting a second point in the graph space; and e) connecting the first and second points in the graph space to define a first area in the graph space that may be usable by a computer to control the injection behavior of the fluid injection device. In some embodiments, the method may also include the step of controlling the fluid injection device using the graph (i.e., injection function).

Thus, the invention may provide a system, computer program product, and method of graphically creating one or more injection protocols that may be used to control the behavior of a fluid injection device.

### BRIEF DESCRIPTION OF THE SEVERAL VIEWS OF THE DRAWING(S)

Having thus described the invention in general terms, reference will now be made to the accompanying drawings, which are not necessarily drawn to scale, and wherein:
FIG. 1 is a diagram of a prior art EMPOWER CT remote control interface having a display region used to both sequentially list and specify phases of constant flow rate and fixed volume separately;
FIG. 2 is a non-limiting description of one alternative embodiment of the present invention showing a diagram of a graphical user interface having a graph space that may be used to graphically plot an injection function in which no control data has been graphically inputted into the graph space;
FIG. 3 is a non-limiting description of one alternative embodiment of the present invention showing a diagram of a graphical user interface in which an injection function has been graphically plotted in the graph space;
FIG. 4 is a non-limiting description of one alternative embodiment of the present invention showing a flow diagram of the process of controlling a fluid injection device in accordance with one embodiment of the present invention;
FIG. 5 is a non-limiting description of one alternative embodiment of the present invention showing a flow diagram of the process of graphically creating an injection function of a graph that may be used to control a fluid injection device;
FIG. 6 is a non-limiting description of one alternative embodiment of the present invention showing a diagram of a continuation of the injection function of FIG. 3 wherein a user has created a second injection function to graphically create an injection protocol that is the combination of the injection function of FIG. 3;
FIG. 7 is a non-limiting description of one alternative embodiment of the present invention showing a flow diagram of the process of graphically creating a second injection function in accordance with one embodiment of the present invention;
FIG. 8 is a non-limiting description of one alternative embodiment of the present invention showing a diagram of the resultant continuation of an injection function of Figure 6, wherein a user has added a third injection function for a second fluid;
FIG. 9 is a non-limiting description of one alternative embodiment of the present invention showing a flow diagram of the process of graphically creating an injection function for a second fluid;
FIG. 10 is a non-limiting description of one alternative embodiment of the present invention showing a diagram wherein a user has graphically plotted an injection function for a second fluid that may be used for concurrent injection of both a first and second fluid;
FIG. 11 is a non-limiting description of one alternative embodiment of the present invention showing a flow diagram of the process of graphically creating an injection function that may be used to control concurrent injection of multiple fluids;
FIG. 12 is a non-limiting description of one alternative embodiment of the present invention showing a diagram of a control console with an injection function that has been generated from sensory input provided by an external device;
FIG. 13 is a non-limiting description of one alternative embodiment of the present invention showing a diagram of the graphical user interface serving as a visible reference for monitoring the adequacy of injection;
FIG. 14 is a non-limiting description of one alternative embodiment of the present invention showing an imaging suite having an injector remote console that includes a graphical user interface that may be used to graphically input injection parameters into a computer system; and
FIG. 15 is a non-limiting description of one alternative embodiment of the present invention showing a diagram of the EMPOWER CT display having a graphical visualization tool superimposed on a graphical interface that may be used to graphically input injection parameters into a computer system.

### DETAILED DESCRIPTION OF THE INVENTION

The present invention now will be described more fully hereinafter with reference to the accompanying drawings, in which some, but not all embodiments of the inventions are shown. The invention may be embodied in many different forms and should not be construed as limited to the embodiments set forth herein. Like numbers refer to like elements throughout. As used herein, the term "exemplary" refers to a non-limiting alternative embodiment of the invention.

In one alternative embodiment, the invention may comprise a graphical visualization tool for controlling the behavior of a medical device including, but not limited to, a device suitable for injecting fluid into a subject. Such device may use a graphical user interface. In some embodiments, the device may include, but is not limited to, a fluid injection function, communication function, extravasation function, monitoring function, and the like. In some embodiments, the graphical visualization tool may have a window including, but not limited to a graph space window for inputting and displaying a desired function. Such function may be directed to one or more fluids. For example, the graphical visualization tool may comprise one or more selector icons that may, for example, permit a user to select from one or more fluids for which a user may input a desired injection function. In addition, the graphical visualization tool may comprise one or more selection icons that may, for example, permit a user to navigate within the graph space to create an injection function for a selected fluid.

In one alternative embodiment, a fluid injection device may use the desired injection function to inject a fluid into a subject. In some embodiments, the graph space may comprise a coordinate system having one or more axes including, but not limited to, a three-dimensional or two-dimensional coordinate system, such as a Cartesian coordinate system. In some embodiments, the Cartesian coordinate system may include a first axis that represents the duration of an injection and a second axis that represents the fluid flow rate of an injection. In one alternative embodiment, the graphical visualization tool may include a textual component for displaying a volume of fluid that is to be injected.

In one alternative embodiment the graphical visualization tool may be configured to run on a control console for a fluid injection device. In one alternative embodiment, the control console may be separate from the fluid injection device. In some embodiments, the injection function may be defined by a graphical plot between a first point and second point in the graph space. In one alternative embodiment, one or more injection functions may be combined to define an injection protocol. In yet another alternative embodiment, one or more injection functions for a first fluid may be combined with one or more injection functions for a second fluid, and so on.

In one alternative embodiment, the graphical user interface may include, but is not limited to, a segment selector icon that permits a user to navigate through the graph space to manipulate and/or interact with one or more functions (e.g., injection functions) including, but not limited to, viewing, creating, and/or editing such functions. In one alternative embodiment, the graphical visualization tool may permit a user to input a first injection function comprising a first fluid and a second injection function comprising a second fluid to be graphically plotted in the graph space, wherein the second injection function overlaps at least a portion of the first injection function in the graph space to define an injection protocol for concurrent fluid injection. In yet another alternative embodiment, the graphical user interface may comprise a touch screen. In some embodiments, a user may graphically plot a function using an input device such as a mouse, keyboard, touchpen, or a combination thereof. In one alternative embodiment, the location selection icon may be navigable in the graph space to permit a user to create an injection function. In some embodiments, the graphical visualization tool may be configured to display a first injection function for a first fluid and a second injection function for a second fluid in colors that are different from each other.

In one alternative embodiment, the invention may include a system comprising a fluid injection device, a control unit, such as a computer, operably connected to the fluid injection device, wherein the computer may include computer readable code thereon for enabling a processor to control the fluid injection device and to permit a user to graphically input parameters for controlling one or more device functions. In one alternative embodiment, the input parameters may include one or more fluid injection parameters. In one alternative embodiment, a user may be able to input the fluid injection parameters into the control unit using a graphical user interface independently of the body region of the subject that is be injected. In some embodiments, the system may include a visual display for displaying the graphical user interface and an input device for permitting a user to graphically input the injection parameters on the graphical user interface. In one embodiment, the control unit may use the inputted fluid injection parameters to control the behavior of the fluid injection device. In some embodiments, the control unit may be disposed in a control room of an imaging suite and the fluid injector device may be disposed in an imaging room of the imaging suite.

In some embodiments, the injection parameters may include, but are not limited to, injection flow rate, injection duration, injection volume, fluid media, or combinations thereof. In one alternative embodiment, the input device may include, but is not limited to, a pointer, touch pad, keyboard, or combinations thereof. In some embodiments, a user may graphically input the fluid injection parameters on a first graph displayed on the graphical user interface that represents the desired behavior of the fluid injection device. In one alternative embodiment, the graph may be in a coordinate system having a first axis that represents a duration of an injection and a second axis that represents an injection flow rate. In another alternative embodiment, the graph may be in a coordinate system having a first axis that represents a duration of an injection and a second axis that represents a fluid volume of an injection. In one alternative embodiment, the graphical user interface may be configured to display a second graph that is superimposed on an image of the first graph, and wherein the second graph represents the real time behavior of the fluid injection device.

In one alternative embodiment, the computer readable code may permit a user to graphically input fluid injection parameters for a first fluid and to graphically input fluid injection parameters for a second fluid to be displayed simultaneously on a graph that represents the desired behavior of the fluid injection device. In some embodiments, the graphical user interface may include a fluid selector icon that permits a user to select the one or more fluids for which the injection parameters are to be graphically input into the computer. In some embodiments, the graphical user interface may be adapted to permit a user to select multiple types of fluid to be injected into the subject at multiple times and may also be adapted to permit a user to select and/or program multiple fluid injections, for example, 1 to 2, 2 to 8, and 8 or more.

In yet another alternative embodiment, the graphical user interface may include a location selection icon that permits a user to select a first point in a graph space and a second point in the graph space wherein the position of the first and second points define an area in the graph that corresponds to an injection function. In one embodiment, at least two of the injection parameters may include injection flow rate and injection duration. In one alternative embodiment, a first point and a second point inputted into the graph space may define an area in the graph space that represents a volume of the fluid to be injected.

In some embodiments, the graphical user interface may be configured to display a volume of fluid to be injected by the injection device. In some embodiments, the graphical user interface may include a first fluid selector icon for selecting a first fluid to be injected and a second fluid selector for selecting a second fluid to be injected. In one alternative embodiment, the first selector icon may permit a user to select a contrast solution and the second selector icon may permit a user to select a flushing solution. In one alternative embodiment, the graphical user interface may include a graph space window for inputting and displaying a desired injection function for a fluid, one or more fluid selector icons that permit a user to select a fluid, and a location selection icon that permits a user to navigate within the graph space to create an injection function for a selected fluid, whereby a fluid injection device may use the created injection function to inject a fluid into a subject.

In another alternative embodiment, the invention may include a computer program product comprising a computer usable medium having computer readable program code embodied therein that may be configured to control a fluid injection device. In some embodiments, the computer program product may comprise a computer readable code configured to cause a computer to display a graphical user interface having: 1) a graph space that may define an area for graphically plotting an injection function that may be used by a computer to control the behavior of a fluid injection device, 2) one or more fluid selector icons that may permit a user to select one or more fluids for which to graphically plot an injection function in the graph space, and 3) a location selection icon that may permit a user to navigate within graph space to graphically plot an injection function in the graph space for the selected fluid.

In some embodiments, the computer readable code may be configured to cause a computer to control the fluid injection device using the graphically created injection function. In one alternative embodiment, the graph space may be in a coordinate system wherein a first axis represents time and wherein a second axis represents fluid flow rate. In another alternative embodiment, the graph may be in a coordinate system wherein a first axis represents time and wherein a second axis represents fluid volume. In yet another alternative embodiment, the graph space may be in a coordinate system wherein a first axis represents fluid flow rate and wherein a second axis represents fluid volume. In one alternative embodiment, the computer readable code may be configured to cause the graphical user interface to display a single graph or multiple graphs, for example, 10 or more graphs. In some embodiments, the computer readable code may be configured to cause the graphical user interface to display multiple graph space windows in which one or more functions may be displayed.

In one alternative embodiment, the computer readable code may be configured to cause the graphical user interface to display a first graph that represents a desired behavior of the fluid injection device. In another alternative embodiment, the computer readable code may be configured to cause a computer to display a second graph that represents an actual behavior of the fluid injection device during an injection. In yet another alternative embodiment, the computer readable code may be configured to simultaneously display a third graph wherein third graph displays fluid pressure as a function of time. In some embodiments, the second graph may be superimposed upon the first graph. In some embodiments, the graphical user interface may include a first graph that represents an injection protocol for a first fluid and a second graph that represents an injection protocol for a second fluid. In one alternative embodiment, the computer readable code may be configured to calculate and display a fluid volume to be injected.

In one alternative embodiment, the computer readable code may be configured to permit a user to do one or more of: 1) select a first fluid; 2) select a first point in the graph space using the location selector icon; 3) select a second point in the graph space using the location selector icon, and wherein the computer readable code is configured to connect the first and second points to define an area in the graph space that is usable by a computer to control the injection behavior of the fluid injection device.

In another alternative embodiment, the computer readable code may be configured to permit a user to do one or more of: select a first fluid and select a third point in the graph space using the location selector icon, wherein the computer readable code is configured to connect the second and third points to define a second area in the graph space and thereby define a piecewise injection function. In yet another alternative embodiment, the computer readable code may be configured to permit a user to do one or more of: select a fluid; select a n^{th} point in the graph space using the location selector icon; and select a n^{th}+1 point in the graph space using the location selector icon, wherein the computer readable code is configured to connect the n^{th} point and the n^{th}+1 point to define one or more areas in the graph space that are usable by a computer to control the injection behavior of the fluid injection device.

In some embodiments, the computer readable code may be configured to cause a computer to select a curve style to be used to connect the first point and the second point. In one alternative embodiment, the computer readable code may be configured to cause a computer to instruct the fluid injection device to inject a first fluid at a rate indicated by a curve between a first point and a second point within the graph space.

In one alternative embodiment, the graph may be created using a sensor or external device which may be in communication with the graphical user interface. In another alternative embodiment, the graph may be created using a database. In one alternative embodiment, the computer program product may be running on a control console for the fluid injection device. In another alternative embodiment, the computer program product may be running on a control console for an imaging equipment device. In yet another alternative embodiment, the computer program product may be running on a control console that is configured to control the fluid injection device and an imaging equipment device.

In another alternative embodiment, the invention may include a method for controlling a fluid injection device comprising the steps of: 1) presenting a graphical user interface to a user wherein the graphical user interface includes a graph space window for inputting and displaying an injection function for a fluid; 2) selecting a first fluid; 3) selecting a first point in the graph space; 4) selecting a second point in the graph space; 5) connecting the first and second points in the graph space to define a first area in the graph space that is usable by a computer to control the injection behavior of the fluid injection device; and 6) controlling the fluid injection device using the injection function.

In some embodiments, the method may also include connecting multiple points within the graph space, for example, 2 to 5, 4 to 10, and permutations thereof to define one or more areas in the graph space. In one alternative embodiment the method may also include: selecting a second fluid; selecting a third point in the graph space; and connecting the second and third points in the graph space to define a second area in the graph space that is usable by a computer to control the injection behavior of the fluid injection device. In some embodiments, at least a portion of the second area may be superimposed upon the first area within the graph space. In some embodiments, the graph space may contain one or more injection functions for one or more fluids.

In one alternative embodiment, the step of connecting the points in the graph space may further comprise the step of selecting a curve style to be used to connect the first point and the second point. In some embodiments, the method may comprise inputting an injection function into the graph space using a sensor or external device that is in communication with a control console for the fluid injection device. In one alternative embodiment, the step of selecting a first fluid may include interacting with one or more fluid selector icons that may be present on the graphical user interface. In some embodiments, the steps of selecting the first and second points in the graph space may further comprise interacting with a location selection icon that is presented by the graphical user interface. In another alternative embodiment, the step of controlling the fluid injection device may comprise instructing the fluid injection device to inject the first fluid at a rate indicated by a curve between the first point and the second point.

In one alternative embodiment, the invention is directed to a graphical visualization tool that may be used with an injection system and a method of its use to graphically input one or more injection parameters into a computer whereby the injection behavior of a fluid injection device may be controlled. In one embodiment, the graphical visualization tool may be running on a control console that may be operably connected to a fluid injection device. In another embodiment, the graphical visualization tool may be used to graphically plot an injection protocol for a given fluid. The thus created injection protocol may be used to control the injection of a fluid into a subject.

In one alternative embodiment, the graphical visualization tool may be used in conjunction with an injection system for administering a fluid to a subject. In one embodiment, the fluid injection system may include a fluid injection device that can be used to administer an effective dosage of a fluid, such as contrast medium, and a control interface that is operatively connected to the fluid injection device. The injection system may have one or more control interfaces. The control interface may send and receive data to and from the fluid injection device. In one alternative embodiment, a graphical user interface according to the present invention may be running on a control interface that may be operably connected to.a fluid injection device. The injection device can be any type of injector mechanism that is used to deliver a contrast medium into a patient or subject (e.g., E-Z-EM EMPOWER CT Injector). In one alternative embodiment, the injection system may be used in conjunction with an imaging system. The imaging system may be comprised of an imaging control console, an imaging device or equipment that can be used to monitor and display the contrast medium within a patient or subject, acquire internal images of a patient or subj ect, and to provide other diagnostic data to a control console or storage media. The imaging system may have an imaging interface that may be operatively connected to the imaging equipment. In some embodiments, the injection system and the imaging system may be operatively connected to a common control console.

The term "contrast medium" includes any suitable medium, that can be injected into an individual or subject to highlight and/or identify selected areas of the individual's body. Contrast mediums may include, but are not limited to radio opaque iodinated injectable media, saline media, flush media, and the like, and any combination or mixture thereof. A contrast medium may be used in conjunction with an imaging device that is used to perform medical diagnostic imaging such as CT scans, MRI, PET, ultrasound, etc.

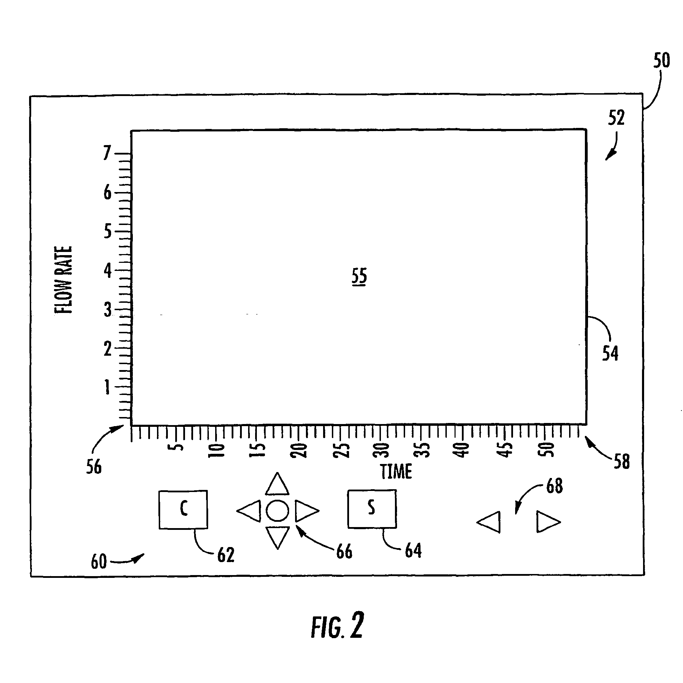

Referring to the figures, FIG. 2 shows a screen display 50 of an alternative embodiment of a graphical visualization tool 52 for controlling the behavior of a fluid injection device using a graphical user interface. FIG. 2 illustrates a graphical visualization tool in which no control data has yet been entered into the graphical user interface. Screen display 50 may include a first display window 54 that may be in the form of a graph space 55 (i.e., injection definition area). In one alternative embodiment, the screen display may be visualized by a user on a visual display, such as computer monitor, LCD monitor, and the like. In some embodiments, the screen display may comprise a touchscreen in which a user may input or select operational instructions via an input action. The graph space may permit a user to graphically plot an injection function that may correspond to an injection protocol. In the context of the invention, the term "injection protocol" refers to the injection of one or more fluids into a subject that may vary in flow rate of fluid over a duration of time.

In some embodiments, an injection protocol may comprise one or more injection functions. In the context of the invention, the term "injection function" refers to a segment of a graph for a selected fluid that has been graphically plotted in the graph space. The injection function of a given fluid may vary in flow rate to deliver the intended volume of fluid over a duration of time. In one alternative embodiment, the graph space may be used to graphically plot injection parameters against each other to create an injection function. For example, in one alternative embodiment, the injection flow rate may be graphically plotted against a desired length of time. In some embodiments, additional injection parameters, such as volume, time, flow rate may be graphically plotted against each other to define an injection function.

In some embodiments, an injection protocol may include multiple injection functions within the graph space, or alternatively, may comprise a single injection function, in which case the injection function is the injection protocol. In using the graphical visualization tool a user may graphically plot multiple injection functions for a given fluid within graph space 55 to create an injection protocol. In some embodiments, one or more injection functions may be combined to create an injection protocol. An injection protocol may be created by combining one or more injection functions that have been graphically plotted in graph space 55.

In some embodiments, the graph space 55 may comprise an injection definition area that corresponds to a Cartesian coordinate system having a first axis 58 and a second axis 56. In some embodiments, axis 58 may represent the duration or length of time and axis 56 may represent the injection flow rate of a fluid that is injected into a subject. In this embodiment, a user may graphically plot an injection function in the graph space wherein the flow rate of the fluid is plotted against the duration of the injection. In an alternative embodiment, axes 56, 58 may represent other injection parameters such as volume versus time as a means of graphically representing an injection function.

The graphical visualization tool 52 may also include a control panel 60 having tools for graphically plotting one or more injection functions in graph space 55. In one alternative embodiment, the control panel 60 may include one or more fluid selector icons 62, 64. Fluid selector icons 62, 64 may permit a user to select a fluid for which an injection function may be created using the graphical visualization tool. In some embodiments, the fluid selector icons may comprise an executable software module that is configured to be displayed within the graphical user interface and may be executable by an input action performed by a user. The number of fluid selector icons that may be available may vary depending upon the application. In some embodiments, the number of fluid selector icons that are available may depend on the number of fluids that the fluid injection device may be able to inject. In some embodiments, the graphical user interface may include one or more pull down menus to access additional fluid selections.

In one alternative embodiment, fluid selector icon 62 may comprise a Fluid 1 Selector button. In some embodiments, the control console for the fluid injection device may include a touch sensitive screen. In this embodiment, the Fluid 1 Selector button may comprise a momentary acting software generated button that appears on the touch screen. In some embodiments, the Fluid 1 Selector button may include a symbol for the type of fluid that is to be injected. Here, the letter "C" is illustrated which may represent a contrast fluid. Applying pressure to the Fluid 1 Selector button may permit a user to use the graphical visualization tool to graphically plot an injection function for a contrast fluid.

In one alternative embodiment, fluid selector icon 64 may comprise a Fluid 2 Selector button. As discussed above, the control console for the fluid injection device may include a touch sensitive screen. In this embodiment, the Fluid 2 Selector button may comprise a momentary acting software generated button that appears on the touch screen. In some embodiments the Fluid 2 Selector button may include a symbol for the type of fluid that is to be injected. Here, the letter "S" is illustrated which may represent a saline fluid used for flushing purposes. Applying pressure to the Fluid 2 Selector button may permit a user to use the graphical visualization tool to graphically plot an injection function for a saline fluid.

In some embodiments, the control panel may also include a location selection icon 66 (i.e., direction pad). Location selection icon 66 may permit a user to use an input device, such as a pointer, keyboard, or similar device, to navigate within graph space 55. In some embodiments, the location selection icon may comprise an executable software module that may be configured to be displayed within the graphical user interface and may be executable by an input action performed by a user. In some embodiments, the location selection icon may comprise a touch sensitive button. In one alternative embodiment, the location selection icon 66 may allow a user to select a first point within the graph space which may correspond to a desired injection parameter. For example, a user may select a first point that corresponds to a desired injection flow rate along axis 58 and a desired point in time during the injection along axis 56. In some embodiments, this first point may correspond to the initial flow rate of the injection and the time at which the injection begins. In the case of a single injection or the first injection in a multi-injection protocol, the initial time may correspond to time zero or the beginning of an injection protocol. Once a first point has been selected using the location selection icon, a second point in graph space 55 may be selected. The second point may correspond to a desired endpoint of the injection function. The graphical visualization tool may then connect the two points to create a graphic plot of the injection function or a portion of an injection protocol for the selected fluid. The graphically created injection protocol may be used to control the injection behavior of the fluid injection device.

In one alternative embodiment, the location selection icon 66 may be in the form of a direction pad. As discussed above, the control console for the fluid injection device may include a touch sensitive screen. In this embodiment, the directional pad may include momentary acting software generated buttons that appear on the control console's touch screen. Depressing direction arrows may be used to graphically position/edit data in the graph space. The direction pad illustrated is generic as it relates to establishing datum in the graph space from which endpoints of graphical segments (i.e., injection functions) may be defined. It is broadly contemplated that such a direction pad could embody additional controls and features to establish graphical segments based upon some mathematical function such as exponentiation, logarithmic, transcendental, and other known mathematical functions.

In some embodiments, a user may graphically plot an injection function in the graph space in several ways. In one alternative embodiment, an injection function may be defined in graph space by selecting a start point (i.e., first point) and end point (i.e., second point) in the graph space. The start point and end points may be selected by using the location selection icon, an input device such as a mouse, touch pad/touch pen, or keyboard, or combinations thereof. Thereafter, the graphical visualization tool may connect the start point and end point to create an injection function. In another embodiment, a user may trace a free-form graph plot in the graph space using an input device, such as mouse or touch pad/touch pen, to define an injection function. In yet another embodiment, the graphic interface may comprise a touch screen and a user may graphically plot an injection function by tracing a free-form plot across the touch screen. In some embodiments, a graphical plot may be created by an external device, such as a monitor, that may input injection parameters.

In one alternative embodiment, the control panel 60 may include a segment selector icon 68 that may permit a user to scroll through graph space to visualize, select, and edit injection functions that have been created in the graph space 55. In some embodiments, the segment selector icon may comprise an executable software module that may be configured to be displayed within the graphical user interface and may be executable by an input action performed by a user. As discussed above, the control console for the fluid injection device may include a touch sensitive screen. In this embodiment, the segment selector icon may comprise a momentary acting software generated button that appears on the touch screen. Segment selector icon may be used to freely select adjacent injection functions that may be defined by the graphical plot of the flow rate versus time.

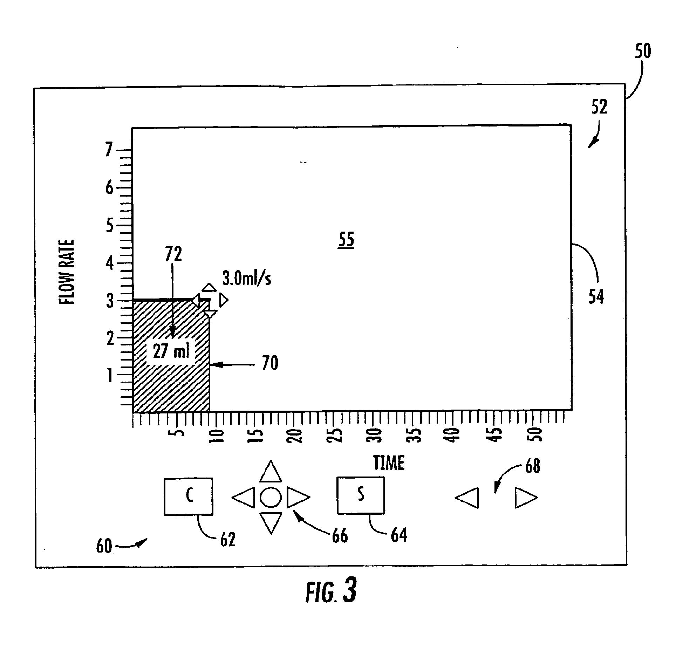

With reference to FIG. 3, a graphical user interface is illustrated in which a user has graphically plotted an injection function 70 in graph space **55**. In the illustrated embodiment, a user has entered an injection function 70 by graphically plotting a line from time 0 to 9 seconds at a constant flow rate of 3.0 ml/s. In some embodiments, the amount of fluid to be injected, 27 ml, may be displayed as textual component 72 in real time during graphical entry of injection function **70.** In some embodiments, the graphical visualization tool may include a software module that calculates the area within the graphical segment to determine the volume of fluid that is to be injected into a subject. In fluid injection protocols where the injection protocol may include multiple injection functions, the graphical visualization tool may be configured to calculate the volume of fluid to be injected for each injection function, total volume injected for an injection protocol comprising multiple injection functions, and combinations thereof.

In some embodiments, to facilitate ease of viewing the on the part of the user, the Flow Rate and Time coordinates defining the injection definition area may automatically refresh their scale during graphical input to maximize viewing of all data relevant to the injection procedure. In yet another embodiment, once either the Time or Flow Rate coordinates defining the injection definition area reach a threshold value, the scale is not adjusted, and a scrolling device may be used instead.

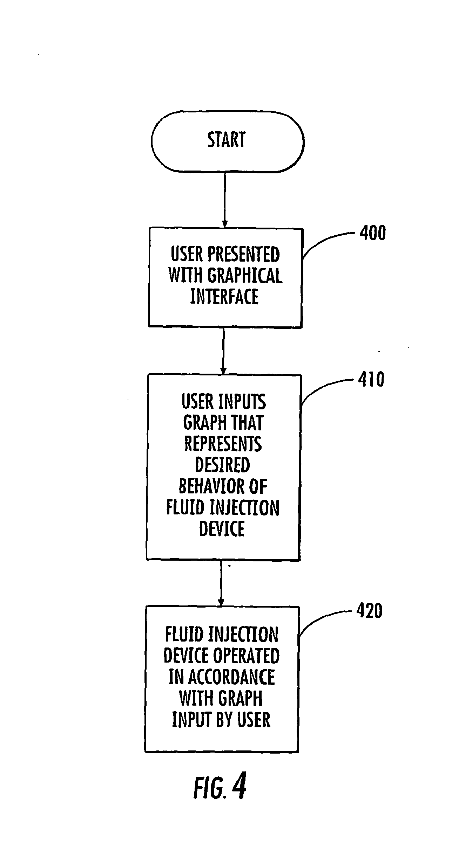

FIG. 4 illustrates a process that may be used to control a fluid injection device in accordance with one embodiment of the present invention. At block 400, a user may be presented with a graphical user interface having a graph space that may be used to define one or more injection functions. At block 410, the user inputs a graph that represents the desired behavior of the fluid injection device. At block 420, the fluid injection device operates in accordance with the graph input by the user.

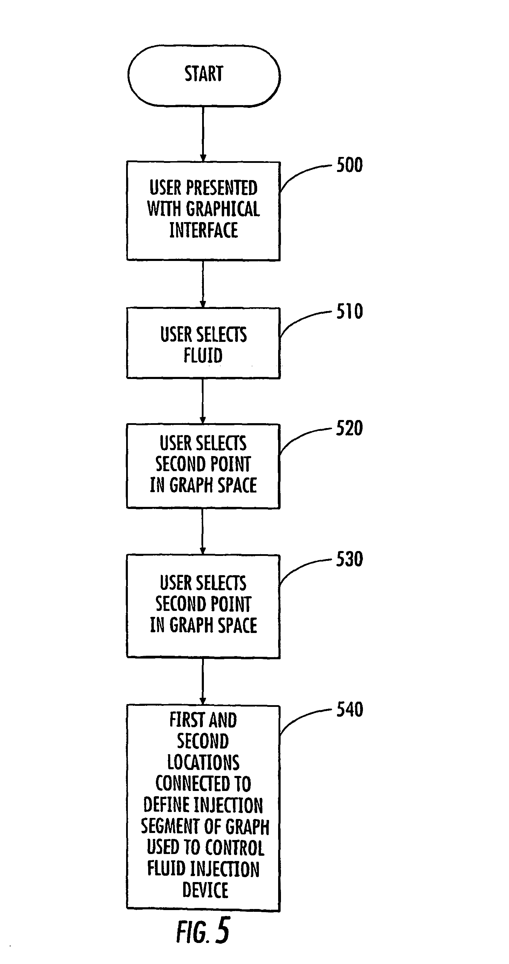

FIG. 5 is a block diagram that illustrates a process that may be used to define an injection function within the graph space whereby the injection function may be used to control a fluid injection device in accordance with one embodiment of the present invention. At block 500, a user may be presented with a graphical user interface having a graphical visualization tool. At block 510, a user may select a fluid to graphically plot in the graph space. At block 520, a user may select a first point within the graph space. At block 530, a user may select a second point in the graph space. At block 540, the graphical visualization tool may connect the first and second points to define an injection function of a graph that may be used to control a fluid injection device. In one embodiment, a user may specify a curve that may be used to connect the first and second points.

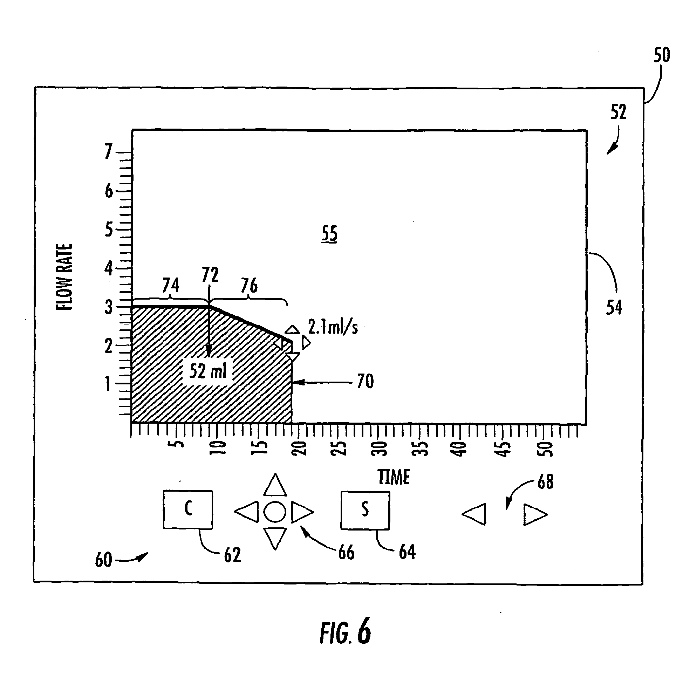

In one alternative embodiment, a user may create multiple injection functions in graph space to create an injection protocol for a given fluid. In one embodiment a user may select an n^{th} point in graph space and an n^{th}+1 point in the graph space. The graphical visualization tool may then connect the n^{th} point and n^{th}+1 point in the graph space to define a piecewise injection protocol. In some embodiments a user may use the location selection icon 66 or the other methods described above to select multiple points in series within graph space to create an injection protocol that may comprise multiple injection functions. In this regard, FIG. 6 illustrates a display screen 50 displaying an injection protocol 70 that is created from graphically plotting two such injection functions within a graph space 50. In the illustration, a user has entered a first injection function 74 by entering a line from time 0 to 9 seconds at a constant flow rate of 3.0 ml/s. The amount of fluid to be injected, 27 ml, may be displayed as textual component (not shown) of injection protocol 70. A user may enter a second injection function 76 by selecting a third point followed by connecting the third point to injection function 74 to define a second injection function 76. In some embodiments, the segment selector icon (see briefly FIG. 3, reference number 68) may permit a user to scroll sequentially from each entered point within the graph space. In the illustration, a user has selected the fluid selector icon a second time and the editing keys (i.e., direction pad and segment selector icon) were used to graphically create a linearly ramp down of the flow rate from 3.0 ml/sec to 2.1 ml/sec over an additional 10 second time period. In one alternative embodiment, the combination of injection functions 74 and 76 may define injection protocol 70. In the illustrated embodiment, both injection functions may comprise the same fluid. In some embodiments the amount of fluid to be injected in the injection protocol may be displayed as textual component 72.

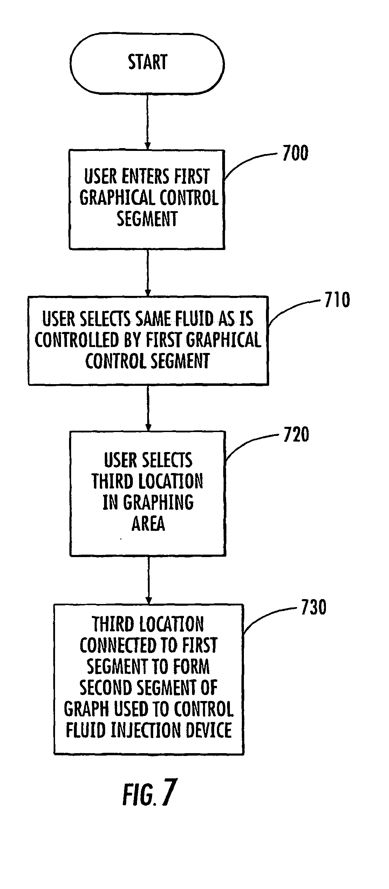

FIG. 7 is a block diagram that illustrates a process that may be used to define a second injection function in accordance with one embodiment of the present invention. At block 700, a user enters a first injection function. At block 710, the user may select the same fluid as is controlled by the first injection function. At block 720, the user selects a third location in the graph space. At block 730, the third location may be connected to the first segment to define a second injection function of a graph that may be used to control a fluid injection device. In other embodiments, the third location may be connected to a fourth location instead of to the first injection function.

Within the functional description of embodiments of the invention, various logical extensions may be implemented. In one alternative embodiment, the graphical user interface may be adapted to create an injection protocol having more than two fluids in a multi-fluid scenario. In some embodiments, the fluid selector icons (see FIG. 2, reference numbers 62, 64) may range from 1 to n, or alternatively, in some embodiments the graphical visualization tool may include a software generated pull down menu arrangement or equivalent. In one alternative embodiment, the fluid selector icons may comprise touch-sensitive buttons. In this embodiment, a user may select a desired fluid by using the touch-sensitive button to scroll through a variety of different fluids. Further, in some embodiments, all available fluids within a multi-fluid arrangement may be freely selectable in any combination and permutation.

In other embodiments, within the creation of a graphical injection function as described here, additional editing means may be provided to the user within the field of the display. In one embodiment, editing capabilities beyond that of linear segments can include but are not limited to mathematical functions (e.g., exponentiation, logarithms, polynomials, transcendental, piecewise, continuous, discontinuous, step, delta, etc.).

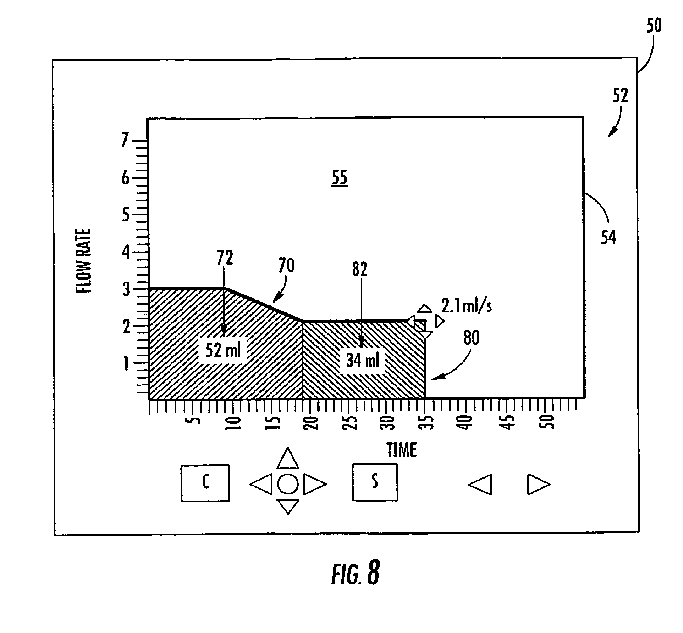

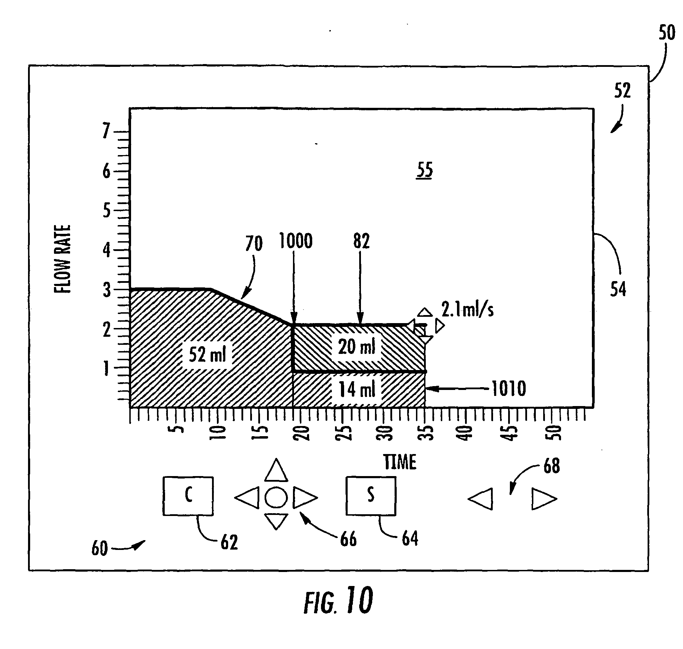

In one alternative embodiment, the graphical user interface may include a graphical visualization tool that may be capable of graphically plotting an injection protocol that includes injection functions for multiple fluids. To create a multi-fluid delivery within the definition of an injection function, a user may select a second fluid for which to define an injection function. With reference to FIG. 8, a graph space 55 is illustrated in which an injection function 70 has been graphically created and a second injection function 80 has been graphically created for a second fluid. The second injection function may be created using the methods discussed above. In some embodiments, the second injection fluid may comprise a saline or other fluid, such as a flushing fluid. **FIG.10** illustrates a continuation of an injection function of **FIG. 8****,** wherein a second injection may be injected at a rate of 2.1 ml/sec for a time period of 16 seconds immediately following that portion of the injection protocol previously defined for contrast delivery in accordance with one embodiment of the present invention. The graphical visualization tool 52 may display a new injection function 80. In some embodiments, the graphical visualization tool may continue to display textual component 72 which may display the total volume of fluid to be injected in the first injection function. In some embodiments, textual component 82 may show the amount of the second fluid that is to be injected.

In some embodiments, the graphical visualization tool may be capable of displaying the injection functions for different fluids in colors or shading that may be used to distinguish the fluids from each other. For example, in one alternative embodiment, an injection function for a contrast fluid may be displayed in a green color whereas an injection function for a saline fluid may be displayed in a red color. Using different colors or shading to visualize different fluids may help a user to readily distinguish between different fluids in the graph space.

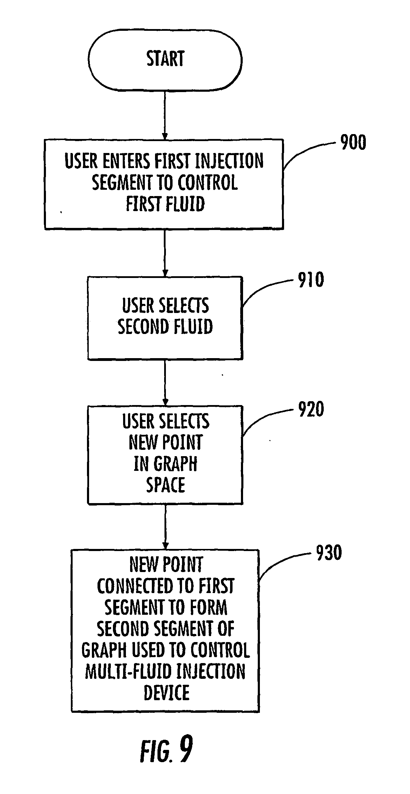

FIG. 9 is a block diagram that illustrates the process of entering an injection function for a second fluid in accordance with one embodiment of the present invention. At block 900, a user enters a first injection function to control a first fluid. At block 910, the user selects a second fluid. At block 920, the user selects a new point in the graphing space. At block 930, the new point may be connected to the first injection function to define a second injection function within graph space that may be used to control a multi-fluid injection device. In other embodiments, the new location is connected to another location instead of to the first graphical segment.

For a multi-fluid arrangement, editing provisions within the display can facilitate the superposition of functions over a common time domain to perform concurrent fluid injection. FIG. 10 illustrates the result of editing FIG. 8 to include a concurrent injection of two fluids, such as contrast and saline, in accordance with one embodiment of the present invention. The graphical visualization tool 52 displays a new fluid injection function 1000. Textual component 70 continues to show the amount of contrast fluid to be injected from 0 to 19 seconds. Likewise, textual component 82 continues to show the amount of the second fluid to be injected from **FIG. 8****.** Now, textual component 1010 may display the amount of a third fluid, which may be the same or different than the first fluid, to be injected concurrently with the second fluid. As shown, in some embodiments, when two fluids are to be injected concurrently, the two fluids may be displayed within graph space 55 as overlapping within the time range shown on the x-axis. As discussed above, the graphical visualization tool may be capable of displaying the injection functions for different fluids in colors or shading that may be used to distinguish the fluids from each other.

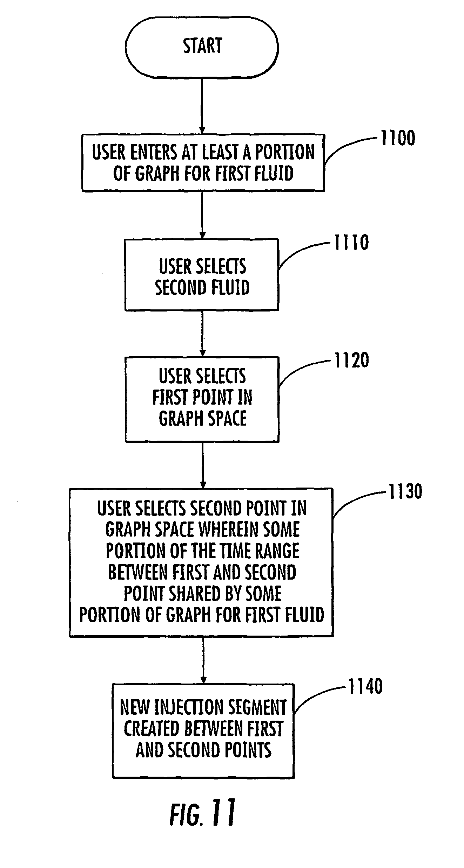

**FIG. 11** is a block diagram that illustrates a process of graphically plotting an injection protocol to control concurrent injection of multiple fluids in accordance with one embodiment of the present invention. At block **1100,** a user enters at least a portion of a graph (injection function) for a first fluid. At block **1110,** the user selects a second fluid. At block **1120**, the user selects a first point in the graphing space. At block **1130,** the user selects a second point in the graphing space wherein some portion of the time range between the first and second points is shared by some portion of the graph for the first fluid. At block **1140,** a new injection function is created between the first and second points.

In one alternative embodiment, the creation of the graphical injection functions, protocols, or combinations thereof are not limited to the editing features within the confines of the control console for the fluid injection device. Within the connectivity, processing and memory capability of the control console, physiological data from a multitude of medical devices with compatible connectivity can be automatically acquired and processed to define a patient specific injection protocol.

**FIG. 12** illustrates a graphical user interface that may be in communication with an external device. In this embodiment, an external device may communication to a control console for the fluid injection device, such as an EMPOWERCT remote control interface. The graphical visualization tool may use the input provided by the external device to generate an injection function and/or injection protocol. In one alternative embodiment, the external device 1200 may include one or more input sensors or monitoring devices that may collect sensor information from one or more medical devices (e.g., a patient monitor, CT scanner, blood analyzer or bio-impedance device). Data from the external device may be communicated via connection **1210** to the control console for the fluid injection device. The graphical visualization tool may create and display fluid injection control graphic **1230** (injection function) based on the data provided by the external device. Connection **1210** may be made using any communications protocol and any physical connection means, including wired and wireless connectivity means. In one embodiment, the shape of fluid injection control graphic 1230 is part or all of the data transmitted via connection **1210.** In another embodiment, the shape of fluid injection control graphic **1230** is determined after the data is communicated via connection 1210. The embodiment of **FIG. 12** is illustrated without a textual component to show the amount of fluid to be injected under control of the graph, but in other similar embodiments, such a textual component is present.

In one alternative embodiment, the present invention prescribes a means whereby a user of a medical injection system specifies fluid delivery through a graphical interface rather than that of a numeric array defined by discreet phases. In this aspect of the invention, the graphical visualization tool may comprise a computer-readable storage medium having computer-executable instructions for displaying the graph space and creating one or more injection functions, protocols, or combinations thereof within the graph space that may be used to control the fluid injection behavior of the fluid injection device. The computer-readable storage medium may be used to upgrade an existing control console, such as an E-Z-EM EMPOWERCT remote control interface. In this embodiment, the graphical visualization tool may replace on a subset or modular level an existing phase method of establishing/defining/programming fluid delivery. This revision will work with all other features and attributes presently defined on the EMPOWERCT Injector Remote Control interface design.

In one embodiment, during the injection procedure, the graphical injection function serves as a visible reference to monitor the adequacy of the injection. FIG. 13 illustrates the graphical user interface serving as a visible reference for monitoring the adequacy of injection in accordance with one embodiment of the present invention. The actual injection progress is graphically mapped using an attribute change to indicate progress (e.g., mapping the actual injection amount in a different color or shading) over the graphically plotted injection multi-fluid graph appearing by the functions defined by different attributes (e.g., illustrating the functions in various colors or shading). In **FIG. 13,** the actual delivery function **1300** departs from the graphically programmed injection function **1310** after about 12 seconds. This represents the injection slowing down from the programmed flow rate most likely due to the occurrence of actual injection pressure attempting to exceed the specified limit at the programmed flow rate. This curtailment of flow rate is commonly known as pressure limiting. In some embodiments, the graphical visualization tool may be configured to display the desired injection volume and the actual injection volume. In the illustration the desired volume is indicated in textual units **1320** and **1330,** and the actual volume is indicated in textual units **1340** and **1350.**

Within the confines of the graphical injection function interface, when, and to what magnitude pressure limiting occurs may be shown. Formerly, in prior art using phase based systems, the user could only ascertain the magnitude of any pressure limiting deficit by scrutinizing quickly changing numeric variables on the display, a difficult or near impossible task for a technologist to perform. Similarly, within the context of this invention, it is reasonably anticipated that an additional variable such as pressure could also be superimposed over, or displayed vertically adjacent to the graphical area time scale. From a phase definition standpoint, pressure as a function of time added as an adjunct to flow rate would provide users with variable pressure limit thresholds over the course of the injection. Extending the injection progress monitoring concept to an ancillary variable such as pressure, the actual pressure on a real-time basis could be correspondingly graphically superimposed during the injection.

As with a data stream coming from an external device to specify a nonlinear injection function prior to injection, within the confines of the data processing capability of the remote control, a real-time data stream **1360** can be output from the control consol to a medical device 1370, which may utilize the injection history information. This time dependent data stream can be incorporated correlated with any other diagnostic or therapeutic data from other devices.

With reference to **FIG. 14,** an alternative embodiment of the present invention depicting a medical imaging suite is shown. As shown in **FIG. 14,** the imaging suite **1400** may include control room **1402** and an imaging equipment room **1404.** The imaging equipment room may comprise an imaging equipment device 1406 and a fluid injection device 1408. The control room **1402** may include a remote control console **1410** that may be operatively connected to the fluid injection device **1408,** and an imaging control console **1412** that may be operatively connected to the imaging equipment device **1406.** In one alternative embodiment, the remote control console **1410** may include a graphical user interface that may be configured to graphically create injection protocols. In some embodiments, the imaging equipment device and the fluid injection device may be in communication with, and operatively controlled by, a common control console (not shown). The control consoles **1410, 1412** can be in communication with devices **1406, 1408** in a wide variety of manners. As shown in **FIG. 14,** the devices **1406, 1408** may each be respectively in communication with their respective control console via communication channels **1414, 1416.** In embodiments where the imaging equipment produces magnetic field, the communication channels between the devices and the control consoles and any additional devices may be adapted to be substantially non-reactive with the magnetic field of the invention. Such substantially non-reactive communication channels include, for example, fiber optic lines, an electromagnetic transmitter/receiver such as an infrared, and the like, and any combination thereof. Additionally, in the embodiments where the imaging equipment produces a magnetic field, the devices such as the injector in the imaging equipment room may comprise a material, such as brass, that is substantially non-reactive with the magnetic field. In other embodiments, the devices in the imaging equipment room may be oriented within the room so that they do not substantially interfere with the imaging equipment.

With reference to **FIG. 15,** an exemplary graphical user interface **1500** for a fluid injection device is illustrated. As shown, graphical user interface **1500** may includes a graphical visualization tool **1502** for graphically inputting injection parameters into the fluid injection device. In one alternative embodiment, the graphical visualization tool **1502** that may permit a user to use a graphical user interface to input injection parameters into a fluid injection device to control the injection behavior of the fluid injection device. In one embodiment, the graphical visualization tool may include a display means for displaying a graph and an input device for inputting one or more desired injection parameters into the graph. In some embodiments, the graph may be used to input a desired injection flow rate and duration of an injection. In this regard, **FIG. 15** illustrates a graphical visualization tool 1502 that is in accordance with one alternative embodiment of the invention. The graphical visualization tool **1502** may include a graph space **1510** in which a user may use an input device, such as a pointer or touch pad, to create an injection function or protocol within graph space **1510** that may represent a desired fluid injection behavior of a fluid to be injected.

In one alternative embodiment the graphical visualization tool may be implemented as a computer graphics display tool through software, firmware, and/or hardware on a computer system. The graphical visualization tool may be provided on any type of graphics workstation, processor, multiprocessor, computer network, stand-alone computer, common control console, remote injector control console, a control console integrated into the fluid injection device, and any other computer graphics processing environment or application that may be operatively connected to a fluid injection device. In some embodiments, one or more injection protocols may be prepared on a separate computer station and uploaded to the fluid injection device or a injection control console at a later time. In one alternative embodiment, the graphical visualization tool may comprise a computer-readable storage medium having computer-executable instructions for displaying the graph space and creating one or more injection functions within the graph space that may be used to control the fluid injection behavior of the fluid injection device. In some embodiments, the injection functions and injection protocols may be stored and recalled for subsequent use.

In one embodiment of the present invention, a graphical user interface is provided for defining an injection function and subsequently controlling a fluid injection device. In one embodiment, a user may control a fluid injection device by inputting a graph. In one embodiment, the graph may be a plot of the desired flow rate versus time. In one embodiment, the graph is a plot of volume versus time. In one embodiment, the graph is a plot of flow rate versus volume.

Referring back to **FIG. 15,** an EMPOWER CT interface display is illustrated as having a graphical visualization tool **1502** superimposed on a graphical user interface. In some embodiments, a user may execute an icon (not shown) on the graphical user interface **1500** that may be used to execute and display graphical visualization tool **1502.** As discussed above, the graphical visualization tool 1502 may include a graph space **1510** in which an injection protocol for one or more fluids may be created. In one alternative embodiment, the graphical visualization tool 1502 may also include one or more fluid selection icons 1520 and 1530 that may permit a user to select one or more fluids for creating an injection function. In some embodiments, the graphical visualization tool 1502 may also include a graph creation icon **1540** (i.e., direction pad) that may permit a user to move within graph space **1510** and pick points in which to create an injection protocol. In one alternative embodiment, a user may create an injection protocol independent of a body region of a subject that is to be imaged. In yet another alternate embodiment, the graphical visualization tool **1502** may also include a graphical selector icon 1550. A graphical selector icon **1550** may permit a user to scroll between individual injection functions that may be included in graph space **1510.**

In one alternative embodiment, a user may create an injection function in graph space **1510** by using graph creation icon **1540** to specify points within graph space **1510.** Additionally, a user may use fluid selection icons **1520** and **1530** to specify which fluid a user may be creating an injection function for in graph space **1510.** In one alternative embodiment, fluid selection icon **1520** may be used to input control instructions related to a contrast fluid. In another alternate embodiment, fluid selection icon 1530 may be used to input control instructions related to a saline solution. Graphical element selector **1550** enables a user to switch between different injection functions within graph space **1510.** Injection functions may be defined by a user or may be generated as a by-product of a mathematical function when the user creates the graph. Within the context of this interface design, it is contemplated that such user interface fluid selectors **1520** and **1530,** graphical element selector 1550 and graph creation tool **1540** could be designed as software generated features on the field of a display as illustrated here, or could be designed as dedicated hardware switches such as on a membrane panel, or a combination thereof. It is also contemplated that the software design for such an interface product may permit direct selection of graphical elements and free form editing of defining points directly in the graphs space 1510 with a standard pointing device such as a mouse or touch screen.

At the discretion of the user, the injector can simply be armed for injection, or graphical injection definition can continue within the confines of the present interface. For example within one embodiment of this invention, the user may select a fluid selector icon a second time to continue adding to the injection function. That portion of the injection function that has already been created may be kept frozen. Upon further utilization of the Direction Pad and Segment Selector the use is able to extend the injection function.

Thus, a method and system for implementing a graphical user interface for a multi-fluid injection device is described in conjunction with one or more specific embodiments. The invention is defined by the following claims and their full scope and equivalents.

Other modifications and other embodiments of the invention set forth herein will come to mind to one skilled in the art to which this invention pertains having the benefit of the teachings presented in the foregoing descriptions and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

Further, throughout the description, where compositions are described as having, including, or comprising specific components, or where processes or methods are described as having, including, or comprising specific steps, it is contemplated that compositions of the present invention may also consist essentially of, or consist of the recited components, and that the processes or methods of the present invention also consist essentially of or consist of the recited steps. Further, it should be understood that the order of steps or order for performing certain actions are immaterial so long as the invention remains operable. Moreover, two or more steps or actions may be conducted simultaneously with respect to the invention disclosed herein.

## Claims

1. A graphical visualization tool (52, 1502) for controlling the behavior of a fluid injection device using a graphical user interface (1500), comprising:
i) a graph space window (54);
ii) one or more fluid selector icons (62, 64) that permit a user to select a fluid; and
iii) a location selection icon (66) that permits a user to navigate within the graph space (55, 1510) to create an injection function (70) for a selected fluid;
wherein
the graph space window (54) is used for defining and displaying an injection protocol comprising at least two injection functions (70) for the fluid,
whereby a fluid injection device (1408) uses the created injection function (70) to inject a fluid into a subject.

2. A fluid injection system comprising:
i) a fluid injection device (1408);
ii) a computer operably connected to the fluid injection device (1408) and having a computer readable code thereon, the code including the graphical visualization tool (52, 1502) of claim 1.
iii) a visual display for displaying the graphical user interface (1500); and
iv) at least one input device for allowing a user to graphically input the injection parameters on the graphical user interface (1500), and wherein the computer uses the fluid injection parameters to control the behavior of the fluid injection device (1408).

3. A graphical visualization tool (52, 1502) according to claim 1 wherein the graph space (55, 1510) comprises a Cartesian coordinate system having a first axis (58) that represents the duration of an injection and a second axis (56) that represents the fluid flow rate of an injection.

4. A graphical visualization tool (52, 1502) according to claims 1 or 3, wherein the graphical visualization tool is configured to run on a control console for a fluid injection device (1408).

5. A graphical visualization tool (52, 1502) according to claim 4, wherein the control console is separate from the fluid injection device (1408).

6. A graphical visualization tool (52, 1502) according to any of the claims 1 and 3 to 5 wherein the graphical user interface (1500) includes a textual component (72, 82) for displaying a volume of fluid that is to be injected.

7. A graphical visualization tool (52, 1502) according to any of the claims 1 and 3 to 6, wherein the injection function (70) is defined by a graphical plot between a first point and second point in the graph space (55, 1510).

8. A graphical visualization tool (52, 1502) according to any of the claims 1 and 3 to 7 wherein any two or more injection function (70) are combined to define the injection protocol.

9. A graphical visualization tool according to any of the claims 1 and 3 to 8 wherein one or more injection functions (70) for a first fluid are combined with one or more injection functions (70) for a second fluid.

10. A graphical visualization tool (52, 1502) according to any of the claims 1 and 3 to 9 wherein the graphical user interface (1500) includes a segment selector icon (68) that permits a user to navigate through the graph space (55, 1510) to view, edit, and create one or more injection functions (70).

11. A graphical visualization tool (52, 1502) according to any of the claims 1 and 3 to 10 wherein a first injection function (74) comprising a first fluid and a second injection function (76, 80) comprising a second fluid are graphically plotted in graph space (55, 1510), and wherein the second injection function (76, 80) overlaps at least a portion of the first injection function (74) in the graph space (55, 1510) to define an injection protocol for concurrent fluid injection.

12. A graphical visualization tool (52, 1502) according to any of the claims 1 and 3 to 11, wherein the graphical user interface (1500) comprises a touch screen.

13. A graphical visualization tool (52, 1502) according to any of claims 3 - 12, wherein the input device is selected from a mouse, keyboard, touchpen, or a combination thereof.

14. A graphical visualization tool (52, 1502) according to any of the claims 1 and 3 to 13, wherein the location selection icon (66) is navigable in the graph space (55, 1510) to create an injection function (70).

15. A graphical visualization tool (52, 1502) according to claim 1, comprising a first injection function (74) for a first fluid and a second injection function (76, 80) for a second fluid and wherein the first and second injection functions (74, 76, 80) are displayed in colors that are different from each other.

16. A fluid injection system according to claim 2, wherein the computer readable code is configured to permit a user to graphically input fluid injection parameters for one or more fluids into the computer using the graphical user interface (1500) independent of a body region of a subject to be injected.

17. A fluid injection system according to claim 2 or 16, wherein the injection parameters include one or more of injection flow rate, injection duration. injection volume, fluid media, or combinations thereof.

18. A graphical visualization tool (52, 1502) according to any of the claims 1 and 3 to 15, wherein a user graphically inputs the fluid injection parameters on a first graph displayed on the graphical user interface (1500) that represents the desired behavior of the fluid injection device (1408).

19. A graphical visualization tool (52, 1502) according to claim18 wherein the graphical user interface (1500) is configured to display a second graph that is superimposed on an image of the first graph, and wherein the second graph represents the real time behavior of the fluid injection device (1408).

20. A computer program product comprising:
a computer usable medium having computer readable program code embodied therein configured to control a fluid injection device (1408), the computer program product comprising
computer readable code configured to cause a computer to display a graphical user interface (1500) having:
i) a graph space (55, 1510);
ii) one or more fluid selector icons (62, 64) that permit a user to select one or more fluids for which to graphically plot an injection function (70) in the graph space (55, 1510); and
iii) a location selection icon (66) that permits a user to navigate within graph space (55, 1510) to graphically plot an injection function (70) in the graph space (55, 1510) for the selected fluid: wherein
the graph space (55, 1510) used for defining an injection protocol comprising at least two injection functions (70),
wherein the graph space (55, 1510) defines an area for graphically plotting a graph of an injection function (70) that is used by a computer to control the behavior of a fluid injection device (1408),
wherein the computer readable code is configured to cause a computer to control the fluid injection device (1408) using the graph.

21. The computer program product of claim 20, wherein the graph space (55, 1510) is in a coordinate system wherein a first axis (58) represents time and wherein a second axis (56) represents fluid flow rate.

22. The computer program product of claim 20, wherein the graph is in a coordinate system wherein a first axis (58) represents time and wherein a second axis. (56) represents fluid volume.

23. The computer program product of any one of claims 20, 21, or 22, wherein the graph space (55, 1510) is in a coordinate system wherein a first axis (58) represents fluid flow rate and wherein a second axis (56) represents fluid volume.

24. The computer program product of claim 23, further comprising computer readable code configured to cause the graphical user interface to display a first graph that represents a desired behavior of the fluid injection device (1408).

25. The computer program product of any one of claims 23 or 24, wherein the computer readable code is configured to cause a computer to display a second graph that represents an actual behavior of the fluid injection device (1408) during an injection.

26. The computer program product of any one of claims 23, 24, or 25, wherein the computer readable code is configured to permit a user to do one or more of:
i. select a first fluid;
ii. select a first point in the graph space (55, 1510) using the location selector icon;
iii. select a second point in the graph space (55, 1510) using the location selector icon, and wherein the computer readable code is configured to connect the first and second points to define an area in the graph space (55, 1510) that is usable by a computer to control the injection behavior of the fluid injection device (1408).

27. The computer program product of claim 26, wherein the computer readable code is configured to permit a user to do one or more of:
iv. select a first fluid; and
v. select a third point in the graph space (55, 1510) using the location selector icon, and wherein the computer readable code is configured to connect the second and third points to define a second area in the graph space (55, 1510) and thereby define a piecewise injection function.

28. The computer program product of claim 27, wherein the computer readable code is configured to permit a user to do one or more of:
vi. select a fluid;
vii. select a n^{th} point in the graph space (55, 1510) using the location selector icon; and
viii. select a n^{th}+1 point in the graph space (55, 1510) using the location selector icon, and wherein the computer readable code is configured to connect the n^{th} point and the n^{th}+1 point to define one or more areas in the graph space (55, 1510) that are usable by a computer to control the injection behavior of the fluid injection device (1408).

29. The computer program product of any one of claims, 23, 24, 25, 26, 27, or 28 wherein the computer readable code is configured to cause a computer to select a curve style to be used to connect the first point and the second point.

30. A method for configuring a fluid injection device (1408) comprising the steps of:
a. presenting a graphical user interface (1500) to a user, the graphical user interface (1500) having a graph space window (54);
b. selecting a first fluid
c. selecting a first point in the graph space (55, 1510) by a location selection icon that permits a user to navigate within the graph space
d. selecting a second point in the graph space (55, 1510) by the location selection icon and
e. connecting the first and second points in the graph space (55, 1510) to define a first area in the graph space (55, 1510) that is usable by a computer to control the injection behavior of the fluid injection device (1408); wherein
the graph space window (54) is used for defining and displaying an injection protocol comprising at least two injection functions (70) for a fluid.

31. A method for configuring a fluid injection device (1408) according to claim 30, further comprising:
selecting a second fluid;
selecting a third point in the graph space (55, 1510); and
connecting the second and third points to define a second area in the graph space (55, 1510) that is usable by a computer to control the injection behavior of the fluid injection device (1408).

32. A method for configuring a fluid injection device (1408) according to claims 30 or 31, further comprising inputting an injection function (70) into the graph space (55, 1510) using a sensor or external device that is in communication with a control console for the fluid injection device (1408).

33. A method for configuring a fluid injection device (1408) according to claims 30, 31, or 32, wherein the step of selecting a first fluid includes interacting with one or more fluid selector icons (62, 64) that are present on the graphical user interface (1500).

## Patentansprüche

1. Graphisches Visualisierungswerkzeug (52, 1502) zum Steuern des Verhaltens einer Fluidinjektionseinrichtung unter Verwendung einer graphischen Benutzeroberfläche (1500), Folgendes umfassend:
i) ein Graphenraumfenster (54);
ii) ein oder mehrere Fluidauswahlicons (62, 64), die einem Benutzer ermöglichen, ein Fluid auszuwählen; und
iii) ein Ortsauswahlicon (66), das einem Benutzer ermöglicht, im Graphenraum (55, 1510) zu navigieren, um eine Injektionsfunktion (70) für ein ausgewähltes Fluid zu erzeugen; worin
das Graphenraumfenster (54) verwendet wird, um ein Injektionsprotokoll zu definieren und anzuzeigen, das mindestens zwei Injektionsfunktionen (70) für dieses Fluid umfasst,
wodurch eine Fluidinjektionseinrichtung (1408) die erzeugte Injektionsfunktion (70) verwendet, um ein Fluid in ein Objekt zu injizieren.

2. Fluidinjektionssystem, Folgendes umfassend:
i) eine Fluidinjektionseinrichtung (1408);
ii) einen Computer, betriebsfähig an die Fluidinjektionseinrichtung (1408) angeschlossen und
mit einem computerlesbaren Code ausgestattet, wobei der Code das graphische Visualisierungswerkzeug (52, 1502) nach Anspruch 1 enthält,
iii) ein Sichtdisplay zum Anzeigen der graphischen Benutzeroberfläche (1500);
und
iv) mindestens eine Eingabeeinrichtung, um einem Benutzer zu ermöglichen, die Injektionsparameter auf der graphischen Benutzeroberfläche (1500) graphisch einzugeben,
und worin der Computer die Fluidinjektionsparameter verwendet, um das Verhalten der Fluidinjektionseinrichtung (1408) zu steuern.

3. Graphisches Visualisierungswerkzeug (52, 1502) nach Anspruch 1, worin der Graphenraum (55, 1510) ein kartesisches Koordinatensystem umfasst, mit einer ersten Achse (58), die die Dauer einer Injektion repräsentiert und einer zweiten Achse (56), die die Fluidflussrate einer Injektion repräsentiert.

4. Graphisches Visualisierungswerkzeug (52, 1502) nach Anspruch 1 oder 3, worin das graphische Visualisierungswerkzeug dazu konfiguriert ist, auf einer Steuerungskonsole für eine Fluidinjektionseinrichtung (1408) abzulaufen.

5. Graphisches Visualisierungswerkzeug (52, 1502) nach Anspruch 4, worin die Steuerungskonsole von der Fluidinjektionseinrichtung (1408) separat ist.

6. Graphisches Visualisierungswerkzeug (52, 1502) nach einem der Ansprüche 1 und 3 bis 5, worin die graphischen Benutzeroberfläche (1500) eine Textkomponente (72, 82) zum Anzeigen eines Volumens von Fluid enthält, das injiziert werden soll.

7. Graphisches Visualisierungswerkzeug (52, 1502) nach einem der Ansprüche 1 und 3 bis 6, worin die Injektionsfunktion (70) durch einen graphischen Plot zwischen einem ersten Punkt und zweiten Punkt im Graphenraum (55, 1510) definiert ist.

8. Graphisches Visualisierungswerkzeug (52, 1502) nach einem der Ansprüche 1 und 3 bis 7, worin zwei oder mehr Injektionsfunktionen (70) kombiniert werden, um das Injektionsprotokoll zu definieren.

9. Graphisches Visualisierungswerkzeug nach einem der Ansprüche 1 und 3 bis 8, worin eine oder mehrere Injektionsfunktionen (70) für ein erstes Fluid mit einer oder mehreren Injektionsfunktionen (70) für ein zweites Fluid kombiniert werden.

10. Graphisches Visualisierungswerkzeug (52, 1502) nach einem der Ansprüche 1 und 3 bis 9, worin die graphische Benutzeroberfläche (1500) ein Segmentauswahlicon (68) enthält, das einem Benutzer ermöglicht, durch den Graphenraum (55, 1510) zu navigieren, um eine oder mehrere Injektionsfunktionen (70) zu betrachten, zu editieren und zu erzeugen.

11. Graphisches Visualisierungswerkzeug (52, 1502) nach einem der Ansprüche 1 und 3 bis 10, worin eine ein erstes Fluid umfassende erste Injektionsfunktion (74) und eine ein zweites Fluid umfassende zweite Injektionsfunktion (76, 80) im Graphenraum (55, 1510) graphisch geplottet werden und worin die zweite Injektionsfunktion (76, 80) mindestens einen Teil der ersten Injektionsfunktion (74) im Graphenraum (55, 1510) überlappt, um ein Injektionsprotokoll für gleichzeitige Fluidinjektion zu definieren.

12. Graphisches Visualisierungswerkzeug (52, 1502) nach einem der Ansprüche 1 und 3 bis 11, worin die graphische Benutzeroberfläche (1500) einen Tastbildschirm umfasst.

13. Graphisches Visualisierungswerkzeug (52, 1502) nach einem der Ansprüche 3 bis 12, worin die Eingabeeinrichtung aus einer Maus, einer Tastatur, einem Taststift oder einer Kombination derselben ausgewählt wird.

14. Graphisches Visualisierungswerkzeug (52, 1502) nach einem der Ansprüche 1 und 3 bis 13, worin das Ortsauswahlicon (66) im Graphenraum (55, 1510) navigierbar ist, um eine Injektionsfunktion (70) zu erzeugen.

15. Graphisches Visualisierungswerkzeug (52, 1502) nach Anspruch 1, das eine erste Injektionsfunktion (74) für ein erstes Fluid und eine zweite Injektionsfunktion (76, 80) für ein zweites Fluid umfasst und worin die erste und zweite Injektionsfunktion (74, 76, 80) in Farben angezeigt werden, die von einander verschieden sind.

16. Fluidinjektionssystem nach Anspruch 2, worin der computerlesbare Code dazu konfiguriert ist, einem Benutzer zu ermöglichen, Fluidinjektionsparameter für ein oder mehrere Fluids in den Computer graphisch einzugeben, unter Verwendung der graphischen Benutzeroberfläche (1500) unabhängig von einem Körperbereich eines Objekts, in das injiziert werden soll.

17. Fluidinjektionssystem nach Anspruch 2 oder 16, worin die Injektionsparameter eine oder mehrere von Folgenden enthalten: Injektionsflussrate, Injektionsdauer, Injektionsvolumen, Fluidmedien oder eine Kombination derselben.

18. Graphisches Visualisierungswerkzeug (52, 1502) nach einem der Ansprüche 1 und 3 bis 15, worin ein Benutzer die Fluidinjektionsparameter auf einem ersten Graph graphisch eingibt, der auf der graphischen Benutzeroberfläche (1500) angezeigt wird, die das gewünschte Verhalten der Fluidinjektionseinrichtung (1408) repräsentiert.

19. Graphisches Visualisierungswerkzeug (52, 1502) nach Anspruch 18, worin die graphische Benutzeroberfläche (1500) dazu konfiguriert ist, einen zweiten Graphen anzuzeigen, der auf ein Bild des ersten Graphen superponiert ist, und worin der zweite Graph das Echtzeitverhalten der ersten Fluidinjektionseinrichtung (1408) repräsentiert.

20. Computerprogrammprodukt, Folgendes umfassend:
ein computernutzbares Medium mit darin eingeschlossenem computerlesbarem Programmcode, konfiguriert zum Steuern einer Fluidinjektionseinrichtung (1408), wobei das Computerprogrammprodukt umfasst:
computerlesbaren Code, der konfiguriert ist, um einen Computer zu veranlassen, eine graphische Benutzeroberfläche (1500) anzuzeigen, mit:
i) einem Graphenraum (55, 1510);
ii) einem oder mehreren Fluidauswahlicons (62, 64), die einem Benutzer ermöglichen, ein oder mehrere Fluids auszuwählen, für die eine Injektionsfunktion (70) im Graphenraum (55, 1510) graphisch geplottet werden soll; und
iii) ein Ortsauswahlicon (66), das einem Benutzer ermöglicht, im Graphenraum (55, 1510) zu navigieren, um für das ausgewählte Fluid im Graphenraum (55, 1510) eine Injektionsfunktion (70) graphisch zu plotten; worin
der zum Definieren eines Injektionsprotokolls verwendete Graphenraum (55, 1510) mindestens zwei Injektionsfunktionen (70) umfasst,
worin der Graphenraum (55, 1510) ein Gebiet zum graphischen Plotten eines Graphen einer Injektionsfunktion (70) definiert, die von einem Computer zum Steuern des Verhaltens einer Fluidinjektionseinrichtung (1408) verwendet wird,
worin der computerlesbare Code dazu konfiguriert ist, einen Computer zu veranlassen, die Fluidinjektionseinrichtung (1408) unter Verwendung des Graphen zu steuern.

21. Computerprogrammprodukt nach Anspruch 20, worin der Graphenraum (55, 1510) in einem Koordinatensystem angeordnet ist, worin eine erste Achse (58) Zeit repräsentiert und worin eine zweite Achse (56) Fluidflussrate repräsentiert.

22. Computerprogrammprodukt nach Anspruch 20, worin der Graph in einem Koordinatensystem angeordnet ist, worin eine erste Achse (58) Zeit repräsentiert und worin eine zweite Achse (56) Fluidvolumen repräsentiert.

23. Computerprogrammprodukt nach einem der Ansprüche 20, 21 oder 22, worin der Graphenraum (55, 1510) in einem Koordinatensystem angeordnet ist, worin eine erste Achse (58) Fluidflussrate repräsentiert und worin eine zweite Achse (56) Fluidvolumen repräsentiert.

24. Computerprogrammprodukt nach Anspruch 23, außerdem computerlesbaren Code umfassend, der dazu konfiguriert ist, das grafische Benutzerdisplay zu veranlassen, einen ersten Graphen anzuzeigen, der ein gewünschtes Verhalten der Fluidinjektionseinrichtung (1408) anzeigt.

25. Computerprogrammprodukt nach einem der Ansprüche 23 oder 24, worin der computerlesbare Code dazu konfiguriert ist, einen Computer zu veranlassen, einen zweiten Graphen anzuzeigen, der ein tatsächliches Verhalten der Fluidinterjektionseinrichtung (1408) während einer Injektion repräsentiert.

26. Computerprogrammprodukt nach einem der Ansprüche 23, 24 oder 25, worin der computerlesbare Code dazu konfiguriert ist, einem Benutzer zu ermöglichen, eine oder mehrere von folgenden Aktionen auszuführen:
i. Auswählen eines ersten Fluids;
ii. Auswählen eines ersten Punkts im Graphenraum (55, 1510) unter Verwendung des Ortsauswahlicons;
iii. Auswählen eines zweiten Punkts im Graphenraum (55, 1510) unter Verwendung des Ortsauswahlicons und worin der computerlesbare Code dazu konfiguriert ist, den ersten und zweiten Punkt zu verbinden, um ein Gebiet im Graphenraum (55, 1510) zu definieren, das von einem Computer verwendet werden kann, um das Injektionsverhalten der Fluidinjektionseinrichtung (1408) zu steuern.

27. Computerprogrammprodukt nach Anspruch 26, worin der computerlesbare Code dazu konfiguriert ist, einem Benutzer zu ermöglichen, eine oder mehrere von folgenden Aktionen auszuführen:
iv. Auswählen eines ersten Fluids; und
v. Auswählen eines dritten Punkts im Graphenraum (55, 1510) unter Verwendung des Ortsauswahlicons und worin der computerlesbare Code dazu konfiguriert ist, den zweiten und dritten Punkt zu verbinden, um ein zweites Gebiet im Graphenraum (55, 1510) zu definieren und dadurch eine stückweise Injektionsfunktion zu definieren.

28. Computerprogrammprodukt nach Anspruch 27, worin der computerlesbare Code dazu konfiguriert ist, einem Benutzer zu ermöglichen, eine oder mehrere von folgenden Aktionen auszuführen:
vi. Auswählen eines Fluids;
vii. Auswählen eines n-ten Punkts im Graphenraum (55, 1510) unter Verwendung des Ortsauswahlicons; und
viii. Auswählen eines (n+1)-ten Punkts im Graphenraum (55, 1510) unter Verwendung des Ortsauswahlicons und worin der computerlesbare Code dazu konfiguriert ist, den n-ten Punkt und den (n+1)-ten Punkt zu verbinden, um ein oder mehrere Gebiete im Graphenraum (55, 1510) zu definieren, die von einem Computer verwendet werden können, um das Injektionsverhalten der Fluidinjektionseinrichtung (1408) zu steuern.

29. Computerprogrammprodukt nach einem der Ansprüche 23, 24, 25, 26, 27 oder 28, worin der computerlesbare Code dazu konfiguriert ist, einen Computer zu veranlassen, einen Kurvenstil auszuwählen, der zum Verbinden des ersten Punkts und des zweiten Punkts verwendet werden soll.

30. Verfahren zum Konfigurieren einer Fluidinjektionseinrichtung (1408), die folgenden Schritte umfassend:
a. Bereitstellen einer graphischen Benutzeroberfläche (1500) für einen Benutzer, wobei die graphische Benutzeroberfläche (1500) ein Graphenraumfenster (54) hat;
b. Auswählen eines ersten Fluids;
c. Auswählen eines ersten Punkts im Graphenraum (55, 1510) durch ein Ortsauswahlicon, das einem Benutzer ermöglicht, im Graphenraum zu navigieren;
d. Auswählen eines zweiten Punkts im Graphenraum (55, 1510) durch das Ortsauswahlicon; und
e. Verbinden des ersten und zweiten Punkts im Graphenraum (55, 1510), um ein erstes Gebiet im Graphenraum (55, 1510) zu definieren, das von einem Computer verwendet werden kann, um das Injektionsverhalten der Fluidinjektionseinrichtung (1408) zu steuern; worin
das Graphenraumfenster (54) zum Definieren und Anzeigen eines Injektionsprotokolls verwendet wird, das mindestens zwei Injektionsfunktionen (70) für ein Fluid umfasst.

31. Verfahren zum Konfigurieren einer Fluidinjektionseinrichtung (1408) nach Anspruch 30, außerdem umfassend:
Auswählen eines zweiten Fluids;
Auswählen eines dritten Punkts im Graphenraum (55, 1510); und
Verbinden des zweiten und dritten Punkts im Graphenraum (55, 1510), um ein zweites Gebiet im Graphenraum (55, 1510) zu definieren, das von einem Computer verwendet werden kann, um das Injektionsverhalten der Fluidinjektionseinrichtung (1408) zu steuern.

32. Verfahren zum Konfigurieren einer Fluidinjektionseinrichtung (1408) nach Anspruch 30 oder 31, außerdem das Eingeben einer Injektionsfunktion (70) in den Graphenraum (55, 1510) unter Verwendung eines Sensors oder einer externen Einrichtung in Kommunikation mit einer Steuerungskonsole für die Fluidinjektionseinrichtung (1408) umfassend.

33. Verfahren zum Konfigurieren einer Fluidinjektionseinrichtung (1408) nach Anspruch 30, 31 oder 32, worin der Schritt des Auswählens eines ersten Fluids das Zusammenwirken mit einem oder mehreren Fluidauswahlicons (62, 64) enthält, die auf der graphischen Benutzeroberfläche (1500) vorhanden sind.

## Revendications

1. Outil de visualisation graphique (52, 1502) pour commander le comportement d'un dispositif d'injection de fluide en utilisant une interface utilisateur graphique (1500), comprenant :
i) une fenêtre d'espace de graphe (54) ;
ii) une ou plusieurs icônes de sélection de fluide (62, 64) qui permettent à un utilisateur de sélectionner un fluide ; et
iii) une icône de sélection d'emplacement (66) qui permet à un utilisateur de naviguer à l'intérieur de l'espace de graphe (55, 1510) pour créer une fonction d'injection (70) pour un fluide sélectionné ;
dans lequel la fenêtre d'espace de graphe (54) est utilisée pour définir et afficher un protocole d'injection comprenant au moins deux fonctions d'injection (70) pour le fluide,
de manière à ce qu'un dispositif d'injection de fluide (1408) utilise la fonction d'injection (70) créée pour injecter un fluide chez un sujet.

2. Système d'injection de fluide, comprenant :
i) un dispositif d'injection de fluide (1408) ;
ii) un ordinateur connecté de manière opérationnelle au dispositif d'injection de fluide (1408) et comportant du code lisible par ordinateur sur celui-ci, le code comprenant l'outil de visualisation graphique (52, 1502) selon la revendication 1 ;
iii) un affichage visuel pour afficher l'interface utilisateur graphique (1500) ; et
iv) au moins un dispositif de saisie pour permettre à un utilisateur de saisir de manière graphique les paramètres d'injection au niveau de l'interface utilisateur graphique (1500) et dans lequel l'ordinateur utilise les paramètres d'injection de fluide pour commander le comportement du dispositif d'injection de fluide (1408).

3. Outil de visualisation graphique (52, 1502) selon la revendication 1, dans lequel l'espace de graphe (55, 1510) comprend un système de coordonnées cartésiennes présentant un premier axe (58) qui représente la durée d'une injection et un deuxième axe (56) qui représente le débit de fluide d'une injection.

4. Outil de visualisation graphique (52, 1502) selon les revendications 1 à 3, dans lequel l'outil de visualisation graphique est configuré pour s'exécuter sur un pupitre de commande pour un dispositif d'injection de fluide (1408).

5. Outil de visualisation graphique (52, 1502) selon la revendication 4, dans lequel le pupitre de commande est séparé du dispositif d'injection de fluide (1408).

6. Outil de visualisation graphique (52, 1502) selon l'une quelconque des revendications 1 et 3 à 5, dans lequel l'interface utilisateur graphique (1500) comprend un élément textuel (72, 82) pour afficher un volume de fluide à injecter.

7. Outil de visualisation graphique (52, 1502) selon l'une quelconque des revendications 1 et 3 à 6, dans lequel la fonction d'injection (70) est définie par un tracé graphique entre un premier point et un deuxième point dans l'espace de graphe (55, 1510).

8. Outil de visualisation graphique (52, 1502) selon l'une quelconque des revendications 1 et 3 à 7, dans lequel deux ou plusieurs fonctions d'injection (70) quelconques sont combinées pour définir le protocole d'injection.

9. Outil de visualisation graphique selon l'une quelconque des revendications 1 et 3 à 8, dans lequel une ou plusieurs fonctions d'injection (70) pour un premier fluide sont combinées avec une ou plusieurs fonctions d'injection (70) pour un deuxième fluide.

10. Outil de visualisation graphique (52, 1502) selon l'une quelconque des revendications 1 et 3 à 9, dans lequel l'interface utilisateur graphique (1500) comprend une icône de sélection de segment (68) qui permet à un utilisateur de naviguer à travers l'espace de graphe (55, 1510) pour visualiser, éditer et créer une ou plusieurs fonctions d'injection (70).

11. Outil de visualisation graphique (52, 1502) selon l'une quelconque des revendications 1 et 3 à 10, dans lequel une première fonction d'injection (74) comprenant un premier fluide et une deuxième fonction d'injection (76, 80) comprenant un deuxième fluide sont tracés de manière graphique dans l'espace de graphe (55, 1510), et dans lequel la deuxième fonction d'injection (76, 80) recouvre au moins une partie de la première fonction d'injection (74) dans l'espace de graphe (55, 1510) pour définir un protocole d'injection pour une injection de fluide simultanée.

12. Outil de visualisation graphique (52, 1502) selon l'une quelconque des revendications 1 et 3 à 11, dans lequel l'interface utilisateur graphique (1500) comprend un écran tactile.

13. Outil de visualisation graphique (52, 1502) selon l'une quelconque des revendications 3 à 12, dans lequel le dispositif de saisie est sélectionné parmi une souris, un clavier, un stylet ou une combinaison de ceux-ci.

14. Outil de visualisation graphique (52, 1502) selon l'une quelconque des revendications 1 et 3 à 13, dans lequel l'icône de sélection d'emplacement (66) peut naviguer dans l'espace de graphe (55, 1510) pour créer une fonction d'injection (70).

15. Outil de visualisation graphique (52, 1502) selon la revendication 1, comprenant une première fonction d'injection (74) pour un premier fluide et une deuxième fonction d'injection (76, 80) pour un deuxième fluide, et dans lequel les première et deuxième fonctions d'injection (74, 76, 80) sont affichées en couleurs qui sont différentes l'une de l'autre.

16. Système d'injection de fluide selon la revendication 2, dans lequel le code lisible par ordinateur est configuré pour permettre à un utilisateur de saisir de manière graphique des paramètres d'injection de fluide pour un ou plusieurs fluides dans l'ordinateur en utilisant l'interface utilisateur graphique (1500) indépendante d'une région du corps d'un sujet devant recevoir l'injection.

17. Système d'injection de fluide selon les revendications 2 ou 16, dans lequel les paramètres d'injection comprennent un ou plusieurs éléments d'un débit d'injection, d'une durée d'injection, d'un volume d'injection, de milieux fluides ou des combinaisons de ceux-ci.

18. Outil de visualisation graphique (52, 1502) selon l'une quelconque des revendications 1 et 3 à 15, dans lequel un utilisateur saisit de manière graphique les paramètres d'injection de fluide sur un premier graphe affiché sur l'interface utilisateur graphique (1500) qui représente le comportement souhaité du dispositif d'injection de fluide (1408).

19. Outil de visualisation graphique (52, 1502) selon la revendication 18, dans lequel l'interface utilisateur graphique (1500) est configurée pour afficher un deuxième graphe qui est superposé à une image du premier graphe, et dans lequel le deuxième graphe représente le comportement en temps réel du dispositif d'injection de fluide (1408).

20. Produit de programme informatique, comprenant :
un support utilisable par ordinateur sur lequel est réalisé du code programme lisible par ordinateur, configuré pour commander un dispositif d'injection de fluide (1408), le produit de programme informatique comprenant
du code lisible par ordinateur, configuré pour amener un ordinateur à afficher une interface utilisateur graphique (1500) comportant :
i) un espace de graphe (55, 1510) ;
ii) une ou plusieurs icônes de sélection de fluide (62, 64) qui permettent à un utilisateur de sélectionner un ou plusieurs fluides pour lesquels il convient de tracer de manière graphique une fonction d'injection (70) dans l'espace de graphe (55, 1510) ; et
iii) une icône de sélection d'emplacement (66) qui permet à un utilisateur de naviguer à l'intérieur de l'espace de graphe (55, 1510) pour tracer de manière graphique une fonction d'injection (70) dans l'espace de graphe (55, 1510) pour le fluide sélectionné ;
dans lequel l'espace de graphe (55, 1510) utilisé pour définir un protocole d'injection comprend au moins deux fonctions d'injection (70),
dans lequel l'espace de graphe (55, 1510) définit une zone pour tracer de manière graphique un graphe d'une fonction d'injection (70) qui est utilisée par un ordinateur pour commander le comportement d'un dispositif d'injection de fluide (1408),
dans lequel le code lisible par ordinateur est configuré pour amener un ordinateur à commander le dispositif d'injection de fluide (1408) en utilisant le graphe.

21. Produit de programme informatique selon la revendication 20, dans lequel l'espace de graphe (55, 1510) se trouve dans un système de coordonnées dans lequel un premier axe (58) représente le temps et dans lequel un deuxième axe (56) représente le débit de fluide.

22. Produit de programme informatique selon la revendication 20, dans lequel le graphe se trouve dans un système de coordonnées dans lequel un premier axe (58) représente le temps et dans lequel un deuxième axe (56) représente le volume de fluide.

23. Produit de programme informatique selon l'une quelconque des revendications 20, 21 ou 22, dans lequel l'espace de graphe (55, 1510) se trouve dans un système de coordonnées dans lequel un premier axe (58) représente le débit de fluide et dans lequel un deuxième axe (56) représente le volume de fluide.

24. Produit de programme informatique selon la revendication 23, comprenant en outre du code lisible par ordinateur, configuré pour amener l'interface utilisateur graphique à afficher un premier graphe qui représente un comportement souhaité du dispositif d'injection de fluide (1408).

25. Produit de programme informatique selon l'une quelconque des revendications 23 ou 24, dans lequel le code lisible par ordinateur est configuré pour amener un ordinateur à afficher un deuxième graphe qui représente un comportement réel du dispositif d'injection de fluide (1408) pendant une injection.

26. Produit de programme informatique selon l'une quelconque des revendications 23, 24 ou 25, dans lequel le code lisible par ordinateur est configuré pour permettre à un utilisateur d'effectuer une ou plusieurs des étapes consistant à :
i. sélectionner un premier fluide ;
ii. sélectionner un premier point dans l'espace de graphe (55, 1510) en utilisant l'icône de sélection d'emplacement ;
iii. sélectionner un deuxième point dans l'espace de graphe (55, 1510) en utilisant l'icône de sélection d'emplacement, et dans lequel le code lisible par ordinateur est configuré pour relier les premier et deuxième points afin de définir une zone dans l'espace de graphe (55, 1510) qui est utilisable par un ordinateur pour commander le comportement d'injection du dispositif d'injection de fluide (1408).

27. Produit de programme informatique selon la revendication 26, dans lequel le code lisible par ordinateur est configuré pour permettre à un utilisateur d'effectuer une ou plusieurs des étapes consistant à :
iv. sélectionner un premier fluide ; et
v. sélectionner un troisième point dans l'espace de graphe (55, 1510) en utilisant l'icône de sélection d'emplacement, et dans lequel le code lisible par ordinateur est configuré pour relier les deuxième et troisième points afin de définir une deuxième zone dans l'espace de graphe (55, 1510) et définir ainsi une fonction d'injection segmentée.

28. Produit de programme informatique selon la revendication 27, dans lequel le code lisible par ordinateur est configuré pour permettre à un utilisateur d'effectuer une ou plusieurs des étapes consistant à :
vi. sélectionner un fluide ;
vii. sélectionner un point n dans l'espace de graphe (55, 1510) en utilisant l'icône de sélection d'emplacement ; et
viii. sélectionner un point n+1 dans l'espace de graphe (55, 1510) en utilisant l'icône de sélection d'emplacement, et dans lequel le code lisible par ordinateur est configuré pour relier les points n et n+1 afin de définir une ou plusieurs zones dans l'espace de graphe (55, 1510) qui sont utilisables par un ordinateur pour commander le comportement d'injection du dispositif d'injection de fluide (1408).

29. Produit de programme informatique selon l'une quelconque des revendications 23, 24, 25, 26, 27 ou 28, dans lequel le code lisible par ordinateur est configuré pour amener un ordinateur à sélectionner un style de courbe à utiliser pour relier le premier point et le deuxième point.

30. Procédé de configuration d'un dispositif d'injection de fluide (1408), comprenant les étapes consistant à :
a. présenter une interface utilisateur graphique (1500) à un utilisateur, l'interface utilisateur graphique (1500) disposant d'une fenêtre d'espace de graphe (54) ;
b. sélectionner un premier fluide ;
c. sélectionner un premier point dans l'espace de graphe (55, 1510) à l'aide d'une icône de sélection d'emplacement qui permet à un utilisateur de naviguer à l'intérieur de l'espace de graphe ;
d. sélectionner un deuxième point dans l'espace de graphe (55, 1510) à l'aide de l'icône de sélection d'emplacement ; et
e. relier les premier et deuxième points dans l'espace de graphe (55, 1510) afin de définir une première zone dans l'espace de graphe (55, 1510) qui est utilisable par un ordinateur afin de commander le comportement d'injection du dispositif d'injection de fluide (1408) ; dans lequel
la fenêtre d'espace de graphe (54) est utilisée pour définir et afficher un protocole d'injection comprenant au moins deux fonctions d'injection (70) pour un fluide.

31. Procédé de configuration d'un dispositif d'injection de fluide (1408) selon la revendication 30, comprenant en outre les étapes consistant à :
sélectionner un deuxième fluide ;
sélectionner un troisième point dans l'espace de graphe (55, 1510) ; et
relier les deuxième et troisième points afin de définir une deuxième zone dans l'espace de graphe (55, 1510) qui est utilisable par un ordinateur pour commander le comportement d'injection du dispositif d'injection de fluide (1408).

32. Procédé de configuration d'un dispositif d'injection de fluide (1408) selon les revendications 30 ou 31, comprenant en outre la saisie d'une fonction d'injection (70) dans l'espace de graphe (55, 1510) en utilisant un capteur ou un dispositif externe en communication avec un pupitre de commande pour le dispositif d'injection de fluide (1408).

33. Procédé de configuration d'un dispositif d'injection de fluide (1408) selon les revendications 30, 31 ou 32, dans lequel l'étape consistant à sélectionner un premier fluide comprend une interaction avec une ou plusieurs icônes de sélection de fluide (62, 64) qui se trouvent sur l'interface utilisateur graphique (1500).
